# EUROPEAN PATENT APPLICATION

(11) **EP 4 148 740 A1**
(43) Date of publication of application: **15.03.2023**
(21) Application number: 22191048.2
(22) Date of filing: 05.09.2019
(51) Int. Cl.: G16B 30/10, G16B 20/20, G16B 20/50, A01H 1/04

(54) **A METHOD OR SYSTEM FOR IDENTIFICATION OF A CAUSATIVE MUTATION CAUSING A PHENOTYPE OF INTEREST IN A TEST SAMPLE**

(30) Priority: 05.09.2018 AU 2018903304; 12.07.2019 AU 2019902478
(62) Divisional of application: 19766108.5
(71) Applicant: Oxford University Innovation Limited, Botley Oxford OX2 0JB (GB)
(72) Inventor: CHAMPION, Clement, Oxfordshire, OX2 0JB (GB); DOLAN, Liam, Oxfordshire, OX2 0JB (GB)
(74) Representative: Plasseraud IP

(57) **Abstract**

A method for identifying a mutation associated with a phenotype of interest in a non-vascular plant, wherein the method comprises (a) aligning the DNA sequence of a reference DNA sequence and identifying a first set of sequence mismatches between the two sequences; wherein the test sample is from a mutagenized nonvascular plant; (b) aligning the DNA sequence of at least one comparison sample to the reference DNA sequence and identifying a second set of sequence mismatches between the two sequences; (c) filtering the first set of mismatches with respect to the second set of mismatches to identify a subset of mismatches that are unique to the first set of mismatches, wherein the subset of mismatches are candidate mutations for the causative mutation; wherein the test sample is from a non-vascular plant exhibiting the phenotype of interest and wherein the at least one comparison sample is from an independent non-vascular plant of the same genus that does not exhibit the phenotype of interest; and wherein the reference DNA sequence is a known reference sequence for a non-vascular plant of the genus. In addition, a method for identifying a mutation associated with a phenotype of interest in a non-vascular plant, wherein the method comprises a) aligning the DNA sequence of a a reference DNA sequence and identifying a first set of sequence mismatches between the two sequences; wherein the test sample is from a mutagenized non-vascular plant; (b) aligning the DNA sequence of at least one comparison sample to the reference DNA sequence and identifying a second set of sequence mismatches between the two sequences; (c) filtering the first set of mismatches with respect to the second set of sequence mismatches to identify a subset of mismatches that are common to the first and second sets of sequence mismatches wherein the test sample and the comparison sample(s) are from independent non-vascular plants exhibiting the phenotype of interest and wherein the independent non-vascular plants are the same genus; and wherein the reference DNA sequence is a known reference sequence or a non-vascular plant of the genus or a non-vascular plant of the genus.

## Description

### Technical Field

The present invention relates generally to a method or system for identification of a causative mutation causing a phenotype of interest in a test sample.

### Background

Identification of the genotype associated with a phenotype of interest is critical in numerous applications in plant biology. Mutation discovery following a mutagenesis experiment typically involves outcrossing the mutant with a wild type plant, generating bulked wild type and mutant populations and identifying mutations that only occur in the bulk of mutants. Doing so allows the genomes of wild type and mutant to recombine, thus reducing the number of background mutations in the mutated genome and increasing the chance of identifying the causative mutation. However, this preliminary step comes at a cost.

First, the need to outcross represents a qualitative limitation to typical mutation discovery pipelines in that sterile mutants cannot be crossed. Many mutations (whether causative or background mutations) are likely to cause sterility.

Second, the need to outcross represents a quantitative limitation to typical mutation discovery pipelines in that outcrossing requires going through at least (and often more than) one reproductive cycle, which takes time and costs money, thus limiting the throughput of the pipeline.

### Summary

It is an object of the present invention to substantially overcome, or at least ameliorate, one or more disadvantages of existing arrangements.

The work leading to this invention has received funding from the European Research Council under the European Union's Seventh Framework Programme (FP7/2007-2013) / ERC grant agreement n° 250284.

Disclosed are arrangements which seek to address the above problems by bypassing the need to outcross prior to discovery of part of the DNA sequence (causative mutation) of a sample DNA sequence that causes a phenotype of interest in a tested sample.

According to a first aspect of the present disclosure, there is provided a method for identification of a causative mutation causing a phenotype of interest in a test sample, the method comprising the step of selecting comparison samples based on either i) a prediction that the test sample and comparison samples form part of a complementation group and/or ii) a prediction that the comparison samples do not harbour the causative mutation, and the method further comprising the steps of a processor configured to execute computer program code stored in a computer readable medium, the computer program code executing the method of: obtaining sample DNA sequence data associated with the test sample; obtaining reference DNA sequence data associated with a reference sample; obtaining comparison DNA sequence data associated with the comparison samples; determining a first set of mismatched DNA sequence data associated with the sample DNA sequence data and the reference DNA sequence data; determining further sets of mismatched DNA sequence data associated with the comparison DNA sequence data and the reference DNA sequence data; and filtering the first set of mismatched DNA sequence data with respect to the further sets of mismatched DNA sequence data to obtain a set of candidate mismatches that include the causative mutation for identification of the causative mutation from within the candidate mismatches.

According to a second aspect of the present disclosure, there is provided a system for identification of a causative mutation causing a phenotype of interest in a test sample, the system comprising a means to select comparison samples based on either i) a prediction that the test sample and comparison samples form part of a complementation group and/or ii) a prediction that the comparison samples do not harbour the causative mutation, and the system further comprising a processor configured to execute computer program code stored in a computer readable medium, the computer program code configured to: obtain sample DNA sequence data associated with the test sample; obtain reference DNA sequence data associated with a reference sample; obtain comparison DNA sequence data associated with the comparison samples; determine a first set of mismatched DNA sequence data associated with the sample DNA sequence data and the reference DNA sequence data; determine further sets of mismatched DNA sequence data associated with the comparison DNA sequence data and the reference DNA sequence data; and filter the first set of mismatched DNA sequence data with respect to the further sets of mismatched DNA sequence data to obtain a set of candidate mismatches that include the causative mutation for identification of the causative mutation from within the candidate mismatches.

According to a third aspect of the present disclosure, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants exhibiting the phenotype of interest and wherein the independent non-vascular plants are the same genus; and
wherein the reference DNA sequence is a known reference sequence for a non-vascular plant of the genus.

According to a fourth aspect of the present disclosure, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

According to a fifth aspect of the present disclosure, there is provided a method for identifying a mutation associated with a phenotype of interest in a fern, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent ferns exhibiting the phenotype of interest and wherein the independent ferns are the same genus; and
wherein the reference DNA sequence is a known reference sequence for a fern of the genus.

According to a sixth aspect of the present disclosure, there is provided a method for identifying a mutation associated with a phenotype of interest in a fern, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample is from exhibiting the phenotype of interest and wherein the comparison sample is from an independent fern of the same genus that does not exhibit the phenotype of interest; and
wherein the reference DNA sequence is a known reference sequence for a fern of the genus.

According to a seventh aspect of the present disclosure, there is provided a method for identifying a mutation associated with a phenotype of interest in an algae, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent algaes exhibiting the phenotype of interest and wherein the independent algaes are the same genus; and
wherein the reference DNA sequence is a known reference sequence for an algae of the genus.

According to an eighth aspect of the present disclosure, there is provided a method for identifying a mutation associated with a phenotype of interest in an algae, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample is from an algae exhibiting the phenotype of interest and wherein the comparison sample is from an independent algae of the same genus that does not exhibit the phenotype of interest; and
wherein the reference DNA sequence is a known reference sequence for an algae of the genus.

### Brief Description of the Drawings

At least one embodiment of the present invention will now be described with reference to the drawings, in which:
Fig. 1 is a process flow diagram of a method for identification of a causative mutation causing a phenotype of interest in a tested sample, according to embodiments of the present invention;
Fig. 2 forms a schematic block diagram of a computer system upon which arrangements described can be practiced, according to embodiments of the present invention;
Fig 3 is a further process flow diagram of a method for identification of a causative mutation causing a phenotype of interest in a tested sample, according to embodiments of the present invention;
Figures 4-7 show representations of sequence reads compared to reference reads as examples of various processes, according to embodiments of the present invention;
Figure 8: Rhizoid phenotype of two-days-old *Marchantia polymorpha* plants. Wild-type rhizoid phenotype (A), wavy rhizoid phenotype (B). Rhizoids are cells that grow straight in the wild type (A) and wavy in some mutants (B).
Figure 9: Dorsal epidermis phenotype of two-months-old *Marchantia polymorpha* plants. Wild-type epidermis phenotype (A), stretched epidermis phenotype (B). The dorsal epidermis shows air pores (A, arrowed), which in some mutants (B) are stretched.
Figure 10: Performance of non-allelism based mutation discovery pipeline in UV4.32.
   A: Effect of increasing number of non-allelic mutant backgrounds on filtering efficiency. B: Number of UV4.32 mismatches in remaining after each filtering step when using 8 non-allelic UV mutant lines.
Figure 11: Performance of non-allelism based mutation discovery pipeline in chlorsulfuron resistant mutants. Increasing the number of allelic mutant backgrounds improves the filtering efficiency. The left-most scatter box represents the number of total mismatches in a chlorsulfuron resistant mutant line before filtering out mismatches that are also observed in the resequenced wild type genome.

### Detailed Description including Best Mode

Where reference is made in any one or more of the accompanying drawings to steps and/or features, which have the same reference numerals, those steps and/or features have for the purposes of this description the same function(s) or operation(s), unless the contrary intention appears.

It is to be noted that the discussions contained in the "Background" section and that above relating to prior art arrangements relate to discussions of documents or devices which form public knowledge through their respective publication and/or use. Such should not be interpreted as a representation by the present inventor(s) or the patent applicant that such documents or devices in any way form part of the common general knowledge in the art.

The following provides certain definitions of terms used in this document.

Phenotype: a set of observable characteristics of an individual.

Phenotype of interest: A phenotype to be investigated using the methods and systems disclosed herein. The phenotype of interest can be a desirable observable characteristic or trait. In some cases, the phenotype of interest may be a set of observable characteristics that are favourable compared to wild type plants. In some cases, the phenotype of interest may a set of observable characteristics that are different compared to wild type plants. In some cases, the phenotype of interest may be a set of observable characteristics that are favourable compared to wild type algae or ferns. In some cases, the phenotype of interest may a set of observable characteristics that are different compared to wild type algae or ferns.

M0, M1 and M2: M0 denotes a plant population (i.e. the parent population) in a mutagenesis experiment prior to exposure to a mutagen. M1 is the notation used to refer to the same plant population (i.e. the parent population) following exposure to a mutagen. M2 generation refers to its progeny following selfing (i.e. the process of crossing the mutant with itself).

Mismatch: a difference in the sequence of a read (e.g. a portion of a DNA sequence of a tested sample being tested to identify a causative mutation within that tested sample) compared to a portion of a reference DNA sequence the read best aligns to.

Mutation: a physical change in the DNA sequence of a mutagenized plant compared to a reference DNA sequence.

Causative mutation: a mutation causing a phenotype of interest.

Background mutation: a mutation that is not causing the phenotype of interest.

Genome Wide Association Study: The identification of inherited genetic variants associated with risk of disease or a particular trait. This method surveys the entire genome for naturally occurring genetic polymorphisms, typically single nucleotide polymorphisms (SNPs), that occur more frequently in cases (people with the disease or trait being assessed) than in controls (people without the disease or trait).

Haploid/diploid/polyploidy: the ploidy state is the number of sets of chromosomes an organism contains at a given time of its life cycle. A plant may alternate between a haploid state and a diploid state during its lifecycle. A haploid plant is a plant that predominantly spends its lifecycle in the haploid state, i.e. with a single set of chromosomes. A diploid plant is a plant that predominantly spends its lifecycle in the diploid state, i.e. with two sets of chromosomes. A polypoid plant is a plant that predominantly spends its lifecycle in the diploid state, i.e. with more than two sets of chromosomes. Liverworts, for example *Marchantia polymorpha,* is haploid during most of its life cycle, i.e. it contains a single set of chromosomes. By comparison, higher plants such as grasses or dicots are diploid or polyploid, i.e. they have 2 or more sets of chromosomes during the major part of their life cycle.

Herbicide: chemical used to kill or inhibit the plants, plant cells, plant seeds or plant tissue growth.

Hetero/homozygote: Haploid, diploid and polyploid organisms can contain as many versions of a given gene as they have sets of chromosomes. For example, a haploid organism contains only one version, or allele, while a diploid organism can contain either 2 different versions of the same gene (heterozygote) or the 2 identical versions of the same gene (homozygote).

Outcrossing: the process of crossing a polymorphic line with a line of reference (usually the parent line) with the intention of segregating the background mutations apart from the causative mutation.

Investigated mutant: refers to the mutant line in which the causative mutation is to be identified.

Non-vascular plant: A plant lacking a vascular system (xylem and phloem).

Segregation analysis: A statistical technique for fitting formal genetic models to data on expressed trait or disease (phenotypes) in biological family members in order to determine the most likely mode of inheritance for the trait or disease under study. Segregation analysis requires multiple generations of family members in order to determine the inheritance patterns of the phenotype being analysed.

Subtracting mutant: independent mutant line used to computationally remove background mutations from an investigated mutant. These can be independent allelic mutant lines or independent non-allelic mutant lines. A comparison sample is a sample of a subtracting mutant.

Complementation group: a complementation group defines a group of alleles (i.e. versions of a gene) that fail to complement each other. By extension a complementation group may also define a group of mutant lines that harbour alleles that fail to complement each other. That is, no recombination of the parental phenotypes is observed in the progeny from a cross between the mutant lines of the same complementation group. In *Marchantia polymorpha* for example, if two mutant alleles are in the same complementation group, 100% of the F1 plants resulting from a cross between the mutants all display the mutant phenotype. A
complementation group typically identifies a single polymorphic gene (i.e. a gene whose DNA sequence is mutated).

Reference DNA sequence: Reference genome sequence for the plant, algae or fern being investigated. Reference DNA sequences are published on publicly available databases.

The purpose of the herein described process and system is to enable the identification of the causative mutation in a chosen plant sample. That is, the described process and system enables the identification of part of a gene sequence (the causative mutation) in a chosen plant sample.

Li et al., 2016 (Gene discovery by chemical mutagenesis and whole-genome sequencing in Dictyostelium, Genome research 26:1268-1276) describes the use of whole genome sequencing for identification of mutant genes in a Dictyostelium, a slime mould. Dictyostelium is a member of the Mycetozoa (Amoebozoa) phylum and therefore phylogenetically distant to non-vascular plants and algae. Thole et al., 2015 (Next-generation sequencing as a tool to quickly identify causative EMS-generated mutations, Plant Signalling & Behaviour 10:1-4) details the identification of ethyl
methanesulfonate generated mutations in Arabidopsis thaliana using whole genome sequencing in M4 mutants. The present invention is not reliant on fertile mutants thus allowing a wider range of mutants to be detected.

The herein described process and system uses an alternative approach to mutation filtering. Instead of outcrossing, the process and system initially identifies all mutations in the mutant investigated, both background and causative mutations. That is, the described process and system includes steps that identify all parts of the DNA sequence that have mutated when compared to a reference DNA sequence. Some of these mutations are background mutations (not considered useful) and others are causative mutations. In the outcrossing method, the outcrossing steps attempted to reduce the number of background mutations at this stage.

Then the process and system compares them with all mutations from other mutants that are either predicted not to carry the causative mutation of the mutant investigated, or predicted to be part of a complementation group with the tested sample. By using multiple subtracting mutants the power of the filtering is increased to remove background mutations and technical artefacts.

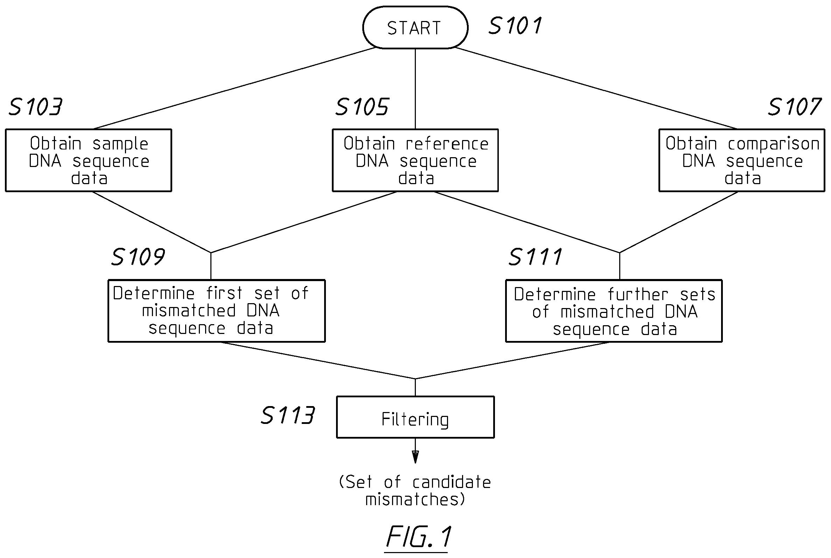

Fig. 1 shows a process flow diagram of a method for identification of a causative mutation causing a phenotype of interest in a tested sample.

In general, the process starts at step S101. At S103, S105 & S107 the sample, reference and comparison (subtracting) DNA sequence data are obtained by the computer program code of the computing system described with reference to Fig. 1. For example, the DNA sequence data may be obtained directly from the DNA sequencing system 1329, or obtained from memory within the computer system, or form memory external to the computer system, or from devices that are external to the computer system via the WAN or LAN.

The sample DNA sequence data is associated with the tested sample. The reference
DNA sequence data is associated with a reference sample. The comparison (or subtracting) DNA sequence data is associated with comparison (or subtracting) samples. Multiple comparison samples are selected, where the selection of each of the multiple comparison samples is made based on either i) a prediction that the tested sample and the selected comparison sample from part of a complementation group or ii) a prediction that the selected comparison sample does not harbour the causative mutation.

At step S109, the process determines a first set of mismatched DNA sequence data associated with the sample DNA sequence data and the reference DNA sequence data.

At step S111, the process determines further sets of mismatched DNA sequence data associated with the comparison DNA sequence data and the reference DNA sequence data.

At step S113, the process carries out various filtering steps based on the type of comparison samples that have been selected (as described below) in order to produce a set of candidate mismatches that include the causative mutation. In particular, at step S113, the first set of mismatched DNA sequence data is filtered with respect to the further sets of mismatched DNA sequence data to obtain a set of candidate mismatches that include the causative mutation for identification of the causative mutation from within the candidate mismatches.

The above steps (and additional steps) are described in more detail herein.

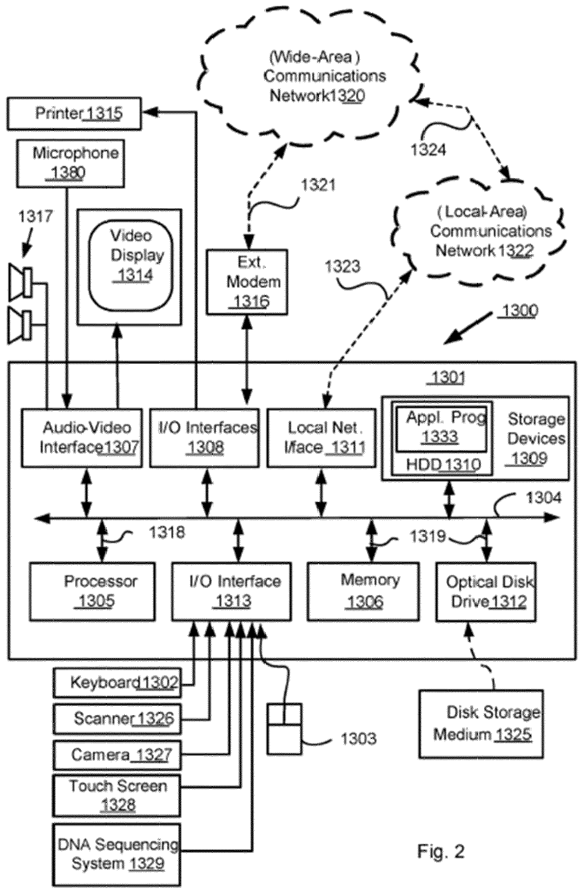

Fig. 2 depicts a computer system 1300, upon which the various arrangements described can be practiced. It will be understood that the computer system will operate in a unique manner once the herein described processes are implemented within the computer system by way of computer program code stored on a computer readable medium. A processor within the computer system is configured to execute the computer program code to carry out the herein described process steps.

As seen in Fig. 2, the computer system 1300 includes: a computer module 1301; input devices such as a keyboard 1302, a mouse pointer device 1303, a scanner 1326, a camera 1327, a touch screen 1328, and a microphone 1380; and output devices including a printer 1315, a display device 1314 and loudspeakers 1317. Further, the computer system may include an input device in the form of a DNA sequencing system 1329. The DNA sequencing system may be, for example the DNA sequencing system may be an Illumina Hiseq series sequencing platform such as a HiSeq 2000 or HiSeq 4000. The output from the DNA sequencing system is two files containing the raw reads, one for each mate of the paired reads. That is, for example, Illumina sequencing platforms are designed to read the extremities of a DNA sequence. Both extremities, also called mates, are linked and they form together a paired read. Two files are therefore produced by Illumina sequencing, one for the left mates and one for the right mates. It will be understood that the invention is not limited to producing paired reads.

It will be understood that the DNA sequencing system 1329 may not be connected directly to the computer system by way of the I/O Interface 1313. For example, the DNA sequencing system 1329 may be connected to a Wide Area network 1320 or Local Area Network 1322.

An external Modulator-Demodulator (Modem) transceiver device 1316 may be used by the computer module 1301 for communicating to and from a communications network 1320 via a connection 1321. The communications network 1320 may be a wide-area network (WAN), such as the Internet, a cellular telecommunications network, or a private WAN. Where the connection 1321 is a telephone line, the modem 1316 may be a high capacity (e.g., cable) connection, or the modem 1316 may be a broadband modem. A wireless modem may also be used for wireless connection to the communications network 1320.

The computer module 1301 typically includes at least one processor unit 1305, and a memory unit 1306. For example, the memory unit 1306 may have semiconductor random access memory (RAM) and semiconductor read only memory (ROM). The computer module 1301 also includes a number of input/output (I/O) interfaces including: an audio-video interface 1307 that couples to the video display 1314, loudspeakers 1317 and microphone 1380; an I/O interface 1313 that couples to the keyboard 1302, mouse 1303, scanner 1326, camera 1327, touch screen 1328 and DNA Sequencing system 1329 (for example) or other human interface device (not illustrated); and an interface 1308 for the external modem 1316 and printer 1315. In some implementations, the modem 1316 may be incorporated within the computer module 1301, for example within the interface 1308. The computer module 1301 also has a local network interface 1311, which permits coupling of the computer system 1300 via a connection 1323 to a local-area communications network 1322, known as a Local Area Network (LAN). As illustrated in Fig. 2, the local communications network 1322 may also couple to the wide network 1320 via a connection 1324, which would typically include a so-called "firewall" device or device of similar functionality. The local network interface 1311 may comprise an Ethernet circuit card, a Bluetooth^{®} wireless arrangement or an IEEE 802.11 wireless arrangement; however, numerous other types of interfaces may be practiced for the interface 1311.

The I/O interfaces 1308 and 1313 may afford either or both of serial and parallel connectivity, the former typically being implemented according to the Universal Serial Bus (USB) standards and having corresponding USB connectors (not illustrated). Storage devices 1309 are provided and typically include a hard disk drive (HDD) 1310. Other storage devices such as a floppy disk drive and a magnetic tape drive (not illustrated) may also be used. An optical disk drive 1312 is typically provided to act as a non-volatile source of data. Portable memory devices, such optical disks (e.g., CD-ROM, DVD, Blu-ray Disc^{™}), USB-RAM, portable, external hard drives, for example, may be used as appropriate sources of data to the system 1300.

The components 1305 to 1313 of the computer module 1301 typically communicate via an interconnected bus 1304 and in a manner that results in a conventional mode of operation of the computer system 1300 known to those in the relevant art. For example, the processor 1305 is coupled to the system bus 1304 using a connection 1318. Likewise, the memory 1306 and optical disk drive 1312 are coupled to the system bus 1304 by connections 1319. Examples of computers on which the described arrangements can be practised include IBM-PC's and compatibles, Apple Mac^{™} or like computer systems.

The methods described herein may be implemented using the computer system 1300 wherein the processes of Fig 1 and associated processes, to be described, may be implemented as one or more software application programs 1333 executable within the computer system 1300. In particular, the steps of the described methods of identifying a causative mutation are effected by instructions 1331 (see Fig. 2) in the software 1333 that are carried out within the computer system 1300. The software instructions 1331 may be formed as one or more code modules, each for performing one or more particular tasks.

The software may be stored in a computer readable medium, including the storage devices described below, for example. The software is loaded into the computer system 1300 from the computer readable medium, and then executed by the computer system 1300. A computer readable medium having such software or computer program recorded on the computer readable medium is a computer program product. The use of the computer program product in the computer system 1300 preferably effects an advantageous apparatus for identifying causative mutations.

The software 1333 is typically stored in the HDD 1310 or the memory 1306. The software is loaded into the computer system 1300 from a computer readable medium, and executed by the computer system 1300. Thus, for example, the software 1333 may be stored on an optically readable disk storage medium (e.g., CD-ROM) 1325 that is read by the optical disk drive 1312. A computer readable medium having such software or computer program recorded on it is a computer program product. The use of the computer program product in the computer system 1300 preferably effects an apparatus for identifying causative mutations.

In some instances, the application programs 1333 may be supplied to the user encoded on one or more CD-ROMs 1325 and read via the corresponding drive 1312, or alternatively may be read by the user from the networks 1320 or 1322. Still further, the software can also be loaded into the computer system 1300 from other computer readable media. Computer readable storage media refers to any non-transitory tangible storage medium that provides recorded instructions and/or data to the computer system 1300 for execution and/or processing. Examples of such storage media include floppy disks, magnetic tape, CD-ROM, DVD, Blu-ray^{™} Disc, a hard disk drive, a ROM or integrated circuit, USB memory, a magnetooptical disk, or a computer readable card such as a PCMCIA card and the like, whether or not such devices are internal or external of the computer module 1301. Examples of transitory or non-tangible computer readable transmission media that may also participate in the provision of software, application programs, instructions and/or data to the computer module 1301 include radio or infra-red transmission channels as well as a network connection to another computer or networked device, and the Internet or Intranets including e-mail transmissions and information recorded on Websites and the like.

The second part of the application programs 1333 and the corresponding code modules mentioned above may be executed to implement one or more graphical user interfaces (GUIs) to be rendered or otherwise represented upon the display 1314. Through manipulation of typically the keyboard 1302 and the mouse 1303, a user of the computer system 1300 and the application may manipulate the interface in a functionally adaptable manner to provide controlling commands and/or input to the applications associated with the GUI(s). Other forms of functionally adaptable user interfaces may also be implemented, such as an audio interface utilizing speech prompts output via the loudspeakers 1317 and user voice commands input via the microphone 1380.

In general, the processor 1305 is given a set of instructions which are executed therein. The processor 1305 waits for a subsequent input, to which the processor 1305 reacts to by executing another set of instructions. Each input may be provided from one or more of a number of sources, including data generated by one or more of the input devices 1302, 1303, data received from an external source across one of the networks 1320, 1302, data retrieved from one of the storage devices 1306, 1309 or data retrieved from a storage medium 1325 inserted into the corresponding reader 1312, all depicted in Fig. 2. The execution of a set of the instructions may in some cases result in output of data. Execution may also involve storing data or variables to the memory 1334.

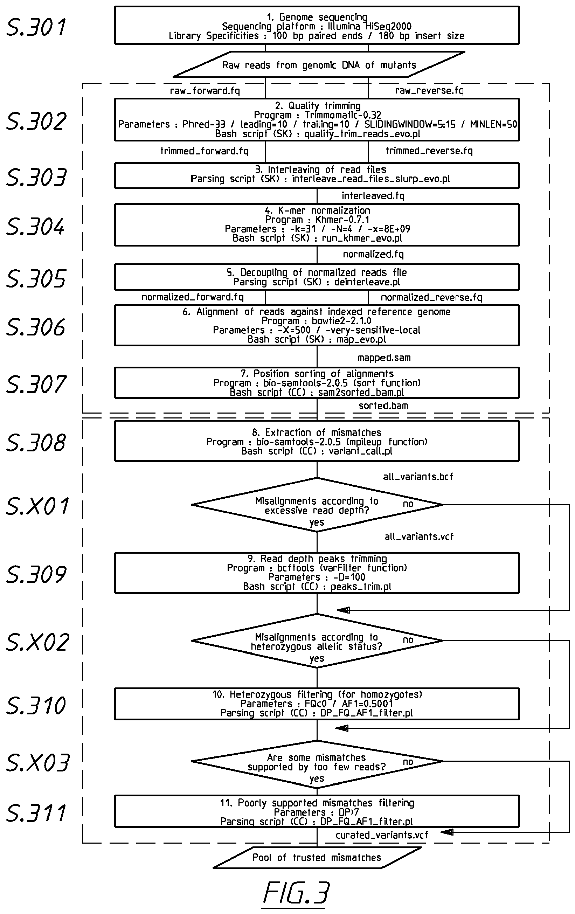

Figure 3 shows a process flow diagram of a method for identification of a causative mutation causing a phenotype of interest in a tested sample.

At step S301, the genomes of the investigated (sample) mutant (and the subtracting (comparison) mutant(s)) are sequenced by the DNA sequencing system to produce tested sample DNA sequence data (and comparison, i.e. subtracting, DNA sequence data). As mentioned above, a DNA sequencing system is used to produce the DNA sequence data for each of the tested sample and the comparison samples. The DNA sequence data includes raw read data that consists of multiple reads, or sequences of text. The DNA sequence data is prepared for analysis after quality-trimming, interleaving and normalization processes are carried out on the DNA sequencing data.

At step S302, the processor carries out the quality trimming process for the tested sample DNA sequence data could, for example, be carried out using a script that calls in any suitable known software program, such as, for example, the program Trimmomatic-0.32. The trimming program trims the Illumina adapters and the part of the reads associated with a poor sequencing quality. Other known processes for carrying out quality trimming may also be used.

At step S303, the processor may carry out an interleaving process, which could, for example, be carried out using any suitable parsing script. For example, where paired reads are obtained by the sequencing system, the parsing script may be used to reunite the two mate reads of all paired reads into a single file.

At step S304, the processor carries out the normalisation process could, for example, be carried out by normalising by 31-mers using a script that calls any suitable known software program, such as, for example, Khmer-0.7.1. In this example, the normalisation program looks at the distribution of k-mers in all reads using a predefined value for k and discards a proportionate amount of reads containing the most frequent k-mers for the reason that they only provide redundant information. This step makes following steps more memory efficient.

At step S305, further, the normalised reads file is de-interleaved or decoupled by the processor using any suitable parsing script that separates the two mate reads of all paired reads in two files. This step is the opposite of the interleaving step. For each paired read, there are two mates identified as belonging to the same paired read. They can either be written in the same file (i.e. interleaved), or in separate files (deinterleaved). The process of going from one to another is merely parsing according to a tagging string that identifies mates as belonging to the same paired read. This tagging originates from the files produced by the sequencing platform and look like XYZ/1 for the mate 1 and XYZ/2 for the mate 2. The software identifies them by text matching and writes the corresponding DNA sequence to either the same files or two separate files.

This step is required for the reads to be processed through the next step.

At steps S306 and S307, the prepared reads of the sample DNA sequence data are aligned by the processor against the reference genome and the alignment files are sorted by the processor by the position of the alignments in the reference genome. Further, the prepared reads of the comparison (i.e. subtracting) DNA sequence data are aligned by the processor against the reference genome and the alignment files are sorted by the processor by the position of the alignments in the reference genome.

For the alignment step at step S306 (for both the sample and the comparison DNA sequence data), the relevant normalised reads are aligned against the reference DNA sequence data using a script that calls any suitable known software program, such as, for example, the program bowtie2-2.1.0. The paired reads are effectively compared to the entire sequence of the genome of the wild type parents of the mutants and the best match is retained. A number of parameters describing the alignment are outputted and written to an alignment file, including the position of the alignment, the confidence that the paired read aligns at this position, the presence, type and detail of mismatches within the alignment, etc.

For the position sorting step at S307 (for both the sample and the comparison DNA sequence data), the relevant alignment file is sorted by the position of the alignments in the reference genome using a script that calls any suitable known software program, such as, for example, the function "sort" from the program bio-samtools-2.0.5.

Mismatches are extracted by the processor at step S308 from the position-sorted alignment files and placed in a candidate mismatch file, if the mismatches are supported by a convincing alignment score. For example, the mismatches are extracted from the positionsorted alignment file using a script that calls in any suitable known software program, such as, for example, the program function mpileup from the program bio-samtools-2.0.5. The mpileup function looks through the position-sorted alignment file looking for the mismatches and writes to a new file all information related to mismatches from reads that have a probability of being correctly aligned above a predefined threshold.

At step S309, regions of the genome where more reads than expected align are excluded by the processor. That is, a *sequencing depth* is defined by the number of sequencing reads from the sample that align against a region of the reference DNA sequence. When sequencing the DNA sequence of a sample, the user may select how many times to sequence the same portion of the DNA sequence. This selection defines the *expected* sequencing depth. For example, aiming for a sequencing depth of 1 would require the sampling system to sequence the entire DNA sequence of the sample a single time. For an expected sequencing depth of 20, the sampling system would sequence 20 times as much of the sample's DNA.

Therefore, as an example, if the *observed* sequencing depth at a defined position is 10, then 10 sequencing reads are aligned to a region of the reference DNA sequence that include this position. If the *expected* sequence depth was 1, this would suggest that 9 of the 10 reads have been aligned against this region of the DNA sequence by mistake. For this reason, the software regards any mismatch in a region of the reference DNA sequence where the *observed* sequencing depth is higher than the *expected* sequencing depth as being the likely consequence of having wrongly aligned reads and thus removes it from set of mismatched data. In other words, the mismatch is regarded as an alignment artefact and not as a candidate mutation and so is discarded or removed from the data set. According to this embodiment, the software uses the mismatch file data and calls a function called varFilter from a program called bcftools. It will be understood that any other suitable software programs may be used to implement this functionality.

The above sequencing depth process at decision step SX01 and step S309, also applies to the further set of comparison DNA sequence data with respect to the reference DNA sequence data.

In other words, to determine the first or further sets of mismatched DNA sequence data, the described method and software may reject at least one region of the sample DNA sequence data that aligns with the reference DNA sequence data based on the actual read depth being over an expected read depth.

Moreover, the system uses the frequency of the mismatch occurrence in the group of reads that align at a position in the genome to filter out alignment artefacts at decision step SX02 and step S310. For example, if the mutant is a diploid species, the expected frequency of the mismatch in the mutant genome is 50%, while in a haploid species it is 100%. If the observed sequencing depth does not match the expected sequencing depth for the defined species, the associated reads are discarded from the set of data. Again, this applies to the sets of data for both the sample and the comparison DNA sequences. Finally, at decision S X03 and step S311, mismatches supported by too few reads are disregarded by the processor by removing the mismatches from the candidate mismatch file. The system further filters the mismatches using biological criteria. The first biological criterion is the specificity of the mismatch to the mutant(s) investigated. There are two main scenarios which may be used either separately or together. For scenario A, the subtracting mutants and the tested sample look or behave alike (i.e. they are phenotypically similar), and/or for scenario B, the independent mutant lines and the tested sample look and behave differently (i.e. they are phenotypically distinct). For scenario A, the comparison (subtracting) samples are selected based on a prediction that the investigated (sample) mutant and the subtracting (comparison) mutants form a complementation group on the basis that they are phenotypically similar. This can optionally be tested by pair-wise crossing if the mutants are not sterile. This prediction step may be termed a "complementation group prediction step" in certain embodiments. Then the system carries out the steps of quality trimming, normalisation, aligning, positioning and sorting (as described above) the mismatched reads (mismatches) for the subtracting lines (i.e. the gene sequence of the hypothesized comparison mutant sample). Then the system compares the set of mismatches of the investigated (sample) mutant to the sets of mismatches of the subtracting (comparison) mutants and retains in a candidate set of mismatches only mismatches that are found both in the investigated mutant and in all subtracting mutants. By retaining the mismatches (compared to the reference sample) that are in both the investigated and subtracting mutant, the chances of one of the mismatches being associated with the causative mutation are much higher. This step therefore reduces the number of candidate mutations and so the amount of further processing that is required to determine the causative mutation. For scenario B, the comparison (subtracting) samples are selected based on a prediction that the subtracting (comparison) mutants do not harbour the causative mutation of the investigated (sample) mutant. This can optionally be tested by pair-wise crossing if the mutants are not sterile. This prediction step may be termed a "comparison causative mutation prediction step" in certain embodiments. Then the system carries out the steps of quality trimming, normalisation, aligning, positioning and sorting (as described above) the mismatched reads (mismatches) for the subtracting comparison lines. Then the system compares the set of mismatches of the investigated (sample) mutant to the sets of mismatches of the subtracting (comparison) mutants and retains in the candidate set of mismatches only mismatches that are specific to the investigated mutant. That is, if the same mismatch in the gene sequence is found in both the subtracting mutant sample and the investigated mutant sample, it is considered that the particular mismatch is not the causative mutation.

The system may perform the process associated with both scenario A and scenario B consecutively or concurrently to increase the power of the entire process.

Finally, standard biological criteria may also be used to discard mismatches in the investigated mutant that are unlikely to be the causative mutations. First, the system may discard mismatches that are not consistent with the mutational signature expected from the mutagenesis method used to create the mutants. That is, non-canonical mismatch filtering may be carried out where the system only retains mismatches in the set of candidate mismatches that do not match the mutational signature of the mutagenesis method used to generate the investigated mutant. Then the system discards mismatches that do not cause a change in the amino acid sequence of the coded protein (i.e. mutations that are in intergenic regions, in untranslated region or in introns). That is, noncoding mismatch filtering may be carried out where the system only retains mismatches in the set of candidate mismatches that cause a change in the amino acid sequence of a protein.

For example, the processor retrieves the coding sequence of the mutated gene (sample) as an input and stores the corresponding translated protein sequence to a temporary array. The system may then repeat the same for the reference coding sequence and push the corresponding protein sequence to the temporary array. Further, the system may then text match both elements of the temporary array and upon the absence of a match, write the associated mismatch to the set of candidate mismatches.

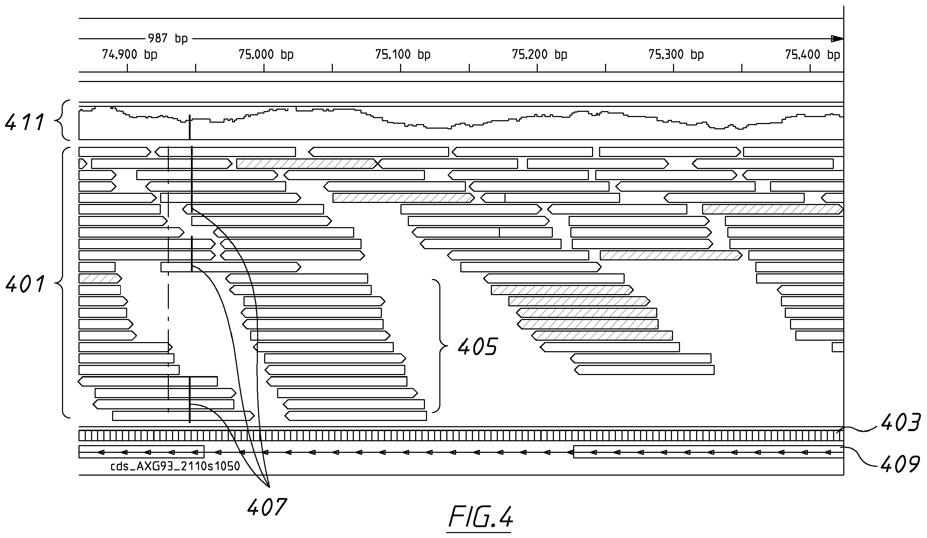

Referring to Figure 4, an example of multiple reads 401 taken from a sample and referenced against a reference DNA sequence 403 is provided. As indicated at 405, the multiple reads from the sample have been aligned and position-sorted with reference to the reference DNA sequence data 403. That is, the reads (of the sample) are represented above the reference sequence that the sample reads correspond to (positionally), or align with.

The vertical line 407 indicates that there is a difference between any read that passes through the line and the reference DNA sequence data at that position. That is, there is a mismatch between the sequence of that read and the reference sequence.

Section 409 indicates the coding sequence of the genes. In the context of this embodiment, a gene is a sequence of DNA that codes for a protein. Parts of the gene carries the information required for the coding of the protein, while the rest do not. The part that carries this information is called the coding sequence of the gene. This is represented visually in 409 with thick black bars (coding sequence) and thin lines (noncoding sequence).

Section 411 indicates the number of reads of the sample at a given position of the reference sequence.

It will be understood that although Figure 4 has been described with reference to multiple reads of sample DNA sequence data and reference DNA sequence data, that the same type of information is also produced with the comparison (subtracting) DNA sequence data and reference DNA sequence data.

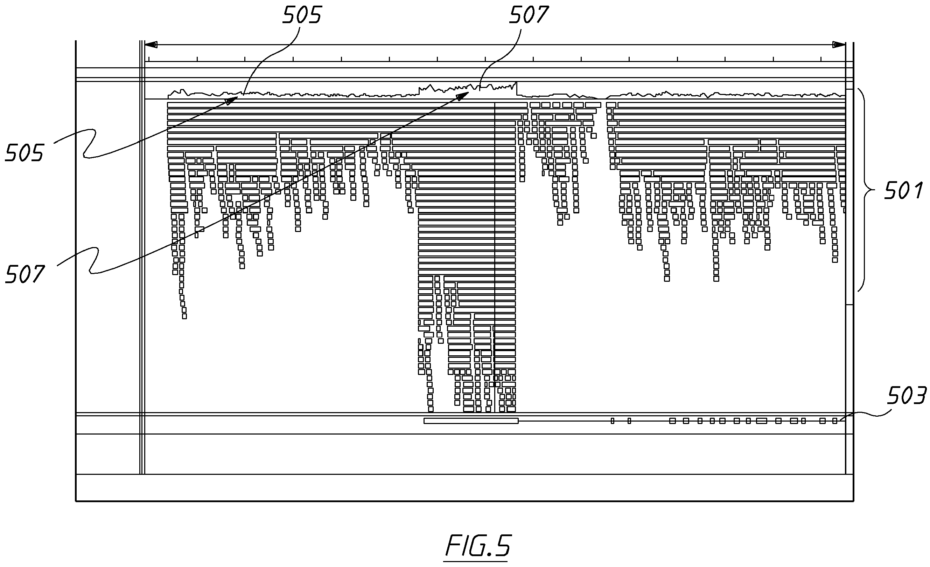

Referring to Figure 5, a further example of multiple reads 501 taken from a sample and referenced against a single read 503 from reference DNA sequence data is provided. In this graphical representation, the observed number of reads of the sample DNA sequence is shown at the associated position relative to the reference sequence. This enables the software to determine whether the expected sequencing depth is consistent with the measured sequencing depth.

At position 505, it can be seen that the observed sequencing depth (i.e. number of reads at this position) is consistent with the expected sequencing depth and so the mismatches in these reads are maintained in the candidate mismatch file. Whereas, at position 507, it can be seen that the observed sequencing depth (i.e. number of reads at this position) is not consistent with the expected sequencing depth and so these reads are not maintained in the candidate mismatch file; that is, they are deleted, removed or discarded from the candidate mismatch file.

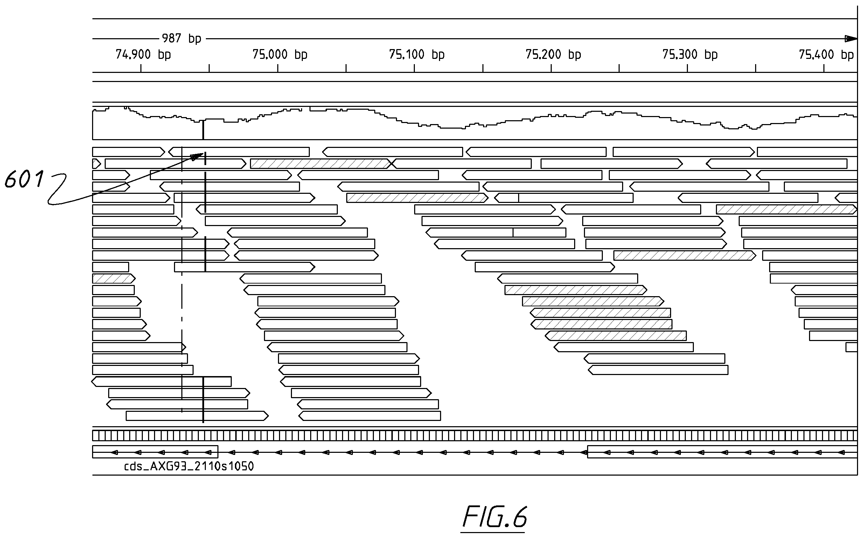

In Figure 6, a graphical representation of determining whether a frequency criterion associated with the expected allele frequency is depicted. In this example, at the position indicated by the arrow 601, the DNA sequence of the sample differs in all the reads at the relevant position with respect to the reference DNA sequence. As such, the allele frequency is 100% and the mismatch data is maintained in the candidate mismatch file as the frequency criterion is met.

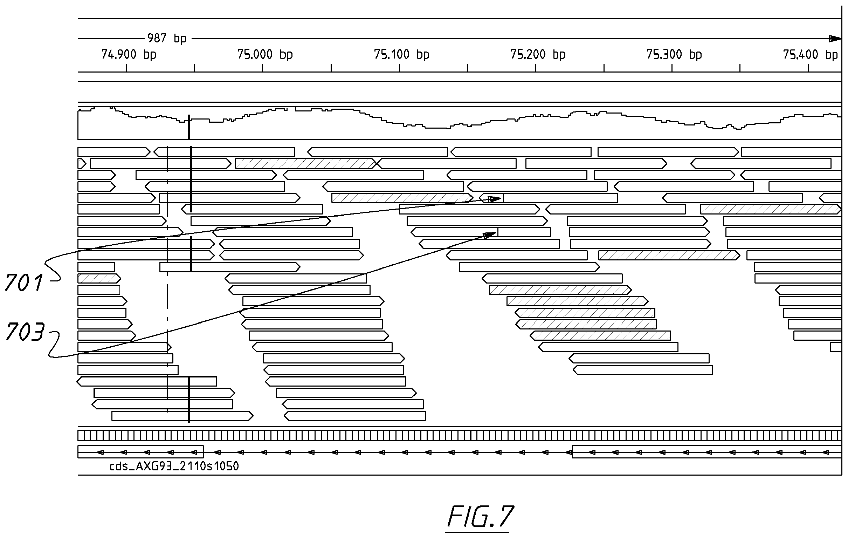

In Figure 7, a graphical representation of determining whether a frequency criterion associated with the expected allele frequency is depicted. In this example, at the position indicated by the arrows 701 and 703, the DNA sequence of the sample differs in these reads at the relevant position with respect to the reference DNA sequence. As such, the allele frequency is not 100% and the mismatch data is not maintained in the candidate mismatch file; that is, it is deleted, removed or discarded from the candidate mismatch file as the frequency criterion is not met.

According to the first, third, fifth and seventh aspects of the disclosure, methods are provided to identify a mutation associated with a phenotype of interest, i.e. a causative mutation. In these aspects, the subtracting mutants and the tested sample look or behave alike (i.e. they are phenotypically similar).

According to a second, fourth, sixth and eighth aspects of the disclosure, methods are provided to identify a mutation associated with a phenotype of interest. In these aspects, the independent mutant lines and the tested sample look and behave differently (i.e. they are phenotypically distinct).

In these aspects, it is not essential to have a prediction or knowledge of the mode of action or biological target associated with the phenotype of interest prior to implementing the method. The method can therefore be used to identify a causative mutation in a plant exhibiting a phenotype of interest wherein the likely genome location of the causative mutation is unknown.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants exhibiting the phenotype of interest and wherein the independent non-vascular plants are the same genus; and
wherein the reference DNA sequence is a known reference sequence for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;
wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, the method further comprises
(b-i) aligning the DNA sequence of at least one additional comparison sample to the reference DNA sequence and identifying a third set of sequence mismatches between the two sequences; wherein the additional comparison sample(s) are from independent non-vascular plants exhibiting the phenotype of interest and wherein the independent non-vascular plants are the same genus; and
wherein (c) further comprises filtering the first set of mismatches with respect to the third set of sequence mismatches to identify a subset of mismatches that are common to the first and third sets of sequence mismatches, wherein the two subsets of mismatches are candidate mutations for the causative mutation.

In one embodiment, the method further comprises
(b-i) aligning the DNA sequence of at least one additional comparison sample to the reference DNA sequence and identifying a third set of sequence mismatches between the two sequences; wherein the additional comparison sample(s) are from independent ferns exhibiting the phenotype of interest and wherein the independent ferns are the same genus; and
wherein (c) further comprises filtering the first set of mismatches with respect to the third set of sequence mismatches to identify a subset of mismatches that are common to the first and third sets of sequence mismatches, wherein the two subsets of mismatches are candidate mutations for the causative mutation.

In one embodiment, the method further comprises
(b-i) aligning the DNA sequence of at least one additional comparison sample to the reference DNA sequence and identifying a third set of sequence mismatches between the two sequences; wherein the additional comparison sample(s) are from independent algae exhibiting the phenotype of interest and wherein the independent algae are the same genus; and
wherein (c) further comprises filtering the first set of mismatches with respect to the third set of sequence mismatches to identify a subset of mismatches that are common to the first and third sets of sequence mismatches, wherein the two subsets of mismatches are candidate mutations for the causative mutation.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences; and
   aligning the DNA sequence of at least one additional comparison sample to the reference DNA sequence and identifying a third set of sequence mismatches between the two sequences,
(c) filtering the first set of mismatches with respect to the second set of mismatches to identify a subset of mismatches that are unique to the first set of mismatches; and

filtering the first set of mismatches with respect to the third set of mismatches to identify a subset of mismatches that are common to the first and third sets of mismatches, wherein the two subsets of mismatches are candidate mutations for the causative mutation and;
wherein the test sample is from a non-vascular plant exhibiting the phenotype of interest and wherein the comparison sample(s) are from an independent non-vascular plant of the same genus that does not exhibit the phenotype of interest and wherein the additional comparison sample(s) are from an independent non-vascular plant of the same genus exhibiting the phenotype of interest; and
wherein the reference DNA sequence is a known reference sequence for a non-vascular plant of the genus.

Exposing a plant or plant population to a mutagen can result in mutations in the plant genome. Similarly, exposing a fern or algae plant or population can result in mutations in their genome. Mutations may occur randomly or may be targeted mutations. Alteration of the genome through the process of mutagenesis may consequently result in an altered phenotype. Numerous mutagens can be used to generate mutant plants, ferns and algae for use in the present invention.

In one embodiment the test sample has been mutagenized. In one embodiment the test sample has been mutagenized by exposure to a mutagen. In one embodiment the test sample and the at least one comparison sample have been mutagenized. In one embodiment the test sample and the at least one comparison sample have been mutagenized by exposure to a mutagen.

In one embodiment, the test sample has been mutagenized wherein mutagenesis is non-naturally occurring. In one embodiment the test sample and the at least one comparison sample have been mutagenized wherein mutagenesis is non-naturally occurring. In one embodiment, the test sample has been mutagenized wherein mutagenesis is experimentally induced. In one embodiment the test sample and the at least one comparison sample have been mutagenized wherein mutagenesis is experimentally induced.

In one embodiment, the mutagen is radiation. In one embodiment, the mutagen is selected from the group consisting of ultra-violet (UV) light, x-ray, gamma rays and neutrons. In one embodiment, the mutagen is UV light, optionally UV-A , UV-B or UV-C light. In one embodiment, the mutagen is UV-B light.

In one embodiment, the mutagen is a chemical agent. In one embodiment, the chemical agent is an alkylating agent, optionally ethyl methanesulfonate (EMS), dimethyl sulphate, sodium azide, methylnitronitrosoguanidine (MNNG). In one embodiment, the chemical agent is a deaminating agent. In one embodiment, the chemical agent is an intercalating agent.

In one embodiment, the mutagen is a transposable element (also known as transposen). A skilled person would understand that there are numerous mutagens for inducing mutagenesis in plants and can be employed in the method of the present invention.

In one embodiment, the test sample and/or at least one comparison sample is nonvascular plant matter that has been mutagenized by exposure to a chemical or physical agent. In one embodiment, the test sample and/or at least one comparison sample is fern biological matter that has been mutagenized by exposure to a chemical or physical agent. In one embodiment, the test sample and/or at least one comparison sample is algae biological matter that has been mutagenized by exposure to a chemical or physical agent.

In one embodiment, the test sample and/or at least one comparison sample is nonvascular plant matter that has been mutagenized by a chemical agent. In one embodiment, the test sample and/or at least one comparison sample is non-vascular plant matter that has been mutagenized by radiation. In one embodiment, the test sample and/or at least one comparison sample is fern biological matter that has been mutagenized by a chemical agent. In one embodiment, the test sample and/or at least one comparison sample is fern biological matter that has been mutagenized by radiation. In one embodiment, the test sample and/or at least one comparison sample is algae biological matter that has been mutagenized by a chemical agent. In one embodiment, the test sample and/or at least one comparison sample is the algae biological matter that has been mutagenized by radiation.

In one embodiment, the method includes the following preliminary steps:
(i) exposing a population of non-vascular plants to a mutagen;
(ii) obtaining a test sample from a mutagenized non-vascular plant exhibiting a phenotype of interest;
(iii) obtaining at least one comparison sample from an independent mutagenized nonvascular plant exhibiting the same phenotype of interest; or
(iii) obtaining at least one comparison sample from an independent mutagenized nonvascular plant not exhibiting the phenotype of interest;
wherein (i) to (iiii) are performed prior to (a).

In one embodiment, the method includes the following preliminary steps:
(i) exposing a population of ferns to a mutagen;
(ii) obtaining a test sample from a mutagenized fern exhibiting a phenotype of interest;
(iii) obtaining at least one comparison sample from an independent mutagenized fern exhibiting the same phenotype of interest; or

(iii) obtaining at least one comparison sample from an independent mutagenized fern not exhibiting the phenotype of interest;
wherein (i) to (iiii) are performed prior to (a).

In one embodiment, the method includes the following preliminary steps:
(i) exposing a population of algae to a mutagen;
(ii) obtaining a test sample from a mutagenized fern exhibiting a phenotype of interest;
(iii) obtaining at least one comparison sample from an independent mutagenized algae exhibiting the same phenotype of interest; or

(iii) obtaining at least one comparison sample from an independent mutagenized algae not exhibiting the phenotype of interest;
wherein (i) to (iiii) are performed prior to (a).

In one embodiment, the method comprises receiving a test and/or at least one comparison sample. In one embodiment, the method comprises receiving the DNA sequences from a test and/or at least one comparison sample. In one embodiment, the method comprises obtaining a test and/or at least one comparison sample. In one embodiment, the method comprises isolating a test and/or at least one comparison sample. In one embodiment, the method comprises isolating the DNA sequences from a test and/or at least one comparison sample.

In one embodiment, the test sample can be the whole plant or a substantial part of the plant. In one embodiment, the test sample can be the whole plant or a substantial part of the fern. In one embodiment, the test sample can be the whole plant or a substantial part of the algae. In one embodiment, the test sample can be a plant protoplast, callus, sporophyte, sporocyte, spore, gemma, gametophyte, sperm, antheridium, rhizoid, zygote or embryo. In one embodiment, the test sample can be non-vasular plant tissue. In one embodiment, the test sample can be fern tissue. In one embodiment, the test sample can be algae tissue.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
   wherein the test sample is from a mutagenized non-vascular plant;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;
wherein the test sample and the comparison sample(s) are from independent non-vascular plants exhibiting the phenotype of interest and wherein the independent non-vascular plants are the same genus; and
wherein the reference DNA sequence is a known reference sequence for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation

wherein the test sample and the comparison sample(s) are from independent M1 generation non-vascular plants that have been exposed to a mutagen and wherein the independent non-vascular plants exhibit the phenotype of interest and wherein the independent non-vascular plants are the same genus; and
wherein the reference DNA sequence is a known reference sequence for a non-vascular plant of the genus.

In one embodiment, there is provided a method for identifying a mutation associated with a phenotype of interest in a non-vascular plant wherein the method comprises:
(i) exposing a population of non-vascular plants to a mutagen;
(ii) obtaining a test sample from a mutagenized non-vascular plant exhibiting a phenotype of interest;
(iii) obtaining at least one comparison sample from an independent mutagenized nonvascular plant exhibiting the same phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are common to the first and second sets of mismatches, wherein the subset of mismatches are candidate mutations for the causative mutation;
wherein the reference DNA sequence is a known reference sequence for the for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
   wherein the test sample is from a mutagenized non-vascular plant;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.
filtering the first set of mismatches with respect to the second set of mismatches to identify a subset of mismatches that are unique to the first set of mismatches, wherein the subset of mismatches are candidate mutations for the causative mutation.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent M1 generation non-vascular plants that have been exposed to a mutagen and wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, there is provided a method for identifying a mutation associated with a phenotype of interest in a non-vascular plant wherein the method comprises:
(i) exposing a population of non-vascular plants to a mutagen;
(ii) obtaining a test sample from a mutagenized non-vascular plant exhibiting a phenotype of interest;
(iii) obtaining at least one comparison sample from an independent mutagenized non-vascular plant not exhibiting the same phenotype of interest; and
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are unique to the first set of mismatches, wherein the subset of mismatches are candidate mutations for the causative mutation;
wherein the reference DNA sequence is a known reference sequence for a non-vascular plant of the genus.

In one embodiment, step (b) comprises aligning the DNA sequence of at least one comparison sample to the reference DNA sequence and identifying a second set of sequence mismatches between the two sequences. At least one comparison sample comprises both one comparison sample, as well as one or more comparison sample (for example, 2, 3, 4, 5, 6, 7, 8, 9, 10, 11, 12, 13, 14, 15 or more comparison samples).

In one embodiment, the step of (b) comprises aligning the DNA sequence of 2, 3, 4, 5, 6, 7, 8, 9, 10, 11, 12, 13, 14, 15 or more comparison samples to the reference DNA sequence and identifying a second set of sequence mismatches between the two sequences. In one embodiment, the method utilises seven or more comparison samples. In the case of multiple comparison samples (i.e. 2 or more), multiple independent comparisons are made. Firstly, between the DNA sequence of the first comparison sample and the reference DNA sequence to obtain a first set of mismatches. Secondly, between the DNA sequence of the second comparison sample and the reference DNA sequence to obtain a second set of mismatches. Thirdly, between the DNA sequence of the third comparison sample and the reference DNA sequence to obtain a third set of mismatches. Similarly, this is performed in relation to the fourth, fifth, sixth, seventh, eighth, ninth, tenth, eleventh, twelfth, thirteenth, fourteenth or fifteenth samples, and can be performed in relation to as many comparison samples as used for the analysis. The first, second, third etc set of mismatches identified in step (b) collectively form the second set of mismatches and are used to filter with respect to the first set of mismatched identified in step (a) of the method. Several tools are known for performing these comparison steps, for example the program vcftools or SnpSift.

The present invention is not reliant on understanding the mode of inheritance of the causative mutation associated with the phenotypic trait of interest. The present invention therefore avoids the time-consuming step, employed in traditional mutagenesis studies, of outcrossing mutants of interest to determine segregation patterns. Furthermore, the present invention is not reliant on associating a phenotype of interest and groups of polymorphisms segregating in the progeny of an M1 mutant with the phenotype of interest. The present invention therefore avoids the time-consuming step, employed in traditional mutagenesis studies, of outcrossing mutants of interest to map causative mutations. In one embodiment, the test sample is a M1 generation mutant. In one embodiment, the test sample and the at least one comparison sample are M1 generation mutants. By virtue of being M1 mutants, the nonvascular plants have not been outcrossed. It is therefore possible to identify causative mutations in M1 mutants that cause sterility since the method does not require the mutants to be crossed to identify a causative mutation.

In one embodiment, the test sample is a M1 or M2 generation mutant. In one embodiment, the test sample and the at least one comparison sample are M1 or M2 generation mutants. In one embodiment, the test sample is a M1, M2 or M3 generation mutant. In one embodiment, the test sample and the at least one comparison sample are M1, M2 or M3 generation mutants.

In one embodiment, the method does not require a step of segregation analysis, complex segregation analysis or bulk segregation analysis to identify a causative mutation associated with a phenotype of interest. In one embodiment, the method does not require a step of fertilisation of the non-vascular plants to identify a causative mutation associated with a phenotype of interest. In one embodiment, the method does not require self-fertilisation, fertilisation, outcrossing, back-crossing or fertilisation with a near-isogenic line of the nonvascular plants to identify a causative mutation associated with a phenotype of interest.

In one embodiment, the method does not require knowledge of the inheritance characteristics for the phenotype of interest to identify the causative mutation. In one embodiment, the method does not comprise the step of determining the inheritance pattern for the phenotype of interest to identify the causative mutation.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of sequence mismatches to identify a subset of mismatches that are common to the first and second sets of sequence mismatches;

wherein the test sample and the comparison sample(s) are from independent non-vascular plants that have been exposed to a mutagen and wherein the independent non-vascular plants exhibit the phenotype of interest and wherein the independent non-vascular plants are the same genus; and
wherein the reference DNA sequence is a known reference sequence for a non-vascular plant of the genus;
wherein the method does not comprise a step of segregation analysis, complex segregation analysis or bulk segregation analysis to identify a causative mutation associated with a phenotype of interest.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants that have been exposed to a mutagen and wherein the independent non-vascular plants exhibit the phenotype of interest and wherein the independent non-vascular plants are the same genus; and
wherein the reference DNA sequence is a known reference sequence for a non-vascular plant of the genus;
the method does not require a step of fertilisation to identify a causative mutation associated with a phenotype of interest.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent non-vascular plants that have been exposed to a mutagen and wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus;
wherein the method does not comprise a step of segregation analysis, complex segregation analysis or bulk segregation analysis to identify a causative mutation associated with a phenotype of interest.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent non-vascular plants that have been exposed to a mutagen and wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus;
wherein the method does not require a step of self-fertilisation to identify a causative mutation associated with a phenotype of interest.

In one embodiment the test sample is biological matter from a haploid plant. In one embodiment the test sample and the at least one comparison sample is biological matter from a haploid plant. In one embodiment the test sample is biological matter from a plant predominantly in the haploid phase during its lifecycle. In one embodiment the test sample and the at least one comparison sample is biological matter from a plant predominantly in the haploid phase during its lifecycle.

In one embodiment, the test sample and/or at least one comparison sample is biological matter from an algae. In one embodiment, the test sample and/or at least one comparison sample is biological matter from a Chlorophyta or streptophyta algae. In one embodiment, the test sample and/or at least one comparison sample is biological matter from
Mesostigmatophyceae, Chlorokybophyceae, Klebsormidiophyceae, Zygnematophyceae, Charophyceae and Coleochaetophyceae.

In one embodiment, the test sample and/or at least one comparison sample is biological matter from an algae of the Chlamydomonas genus. In one embodiment, the test sample and/or at least one comparison sample is biological matter from a Chlamydomonas caudata Wille, Chlamydomonas ehrenbergii Gorozhankin, Chlamydomonas elegans,
Chlamydomonas moewusii, Chlamydomonas nivalis, Chlamydomonas ovoidae or Chlamydomonas reinhardtii algae. In one embodiment, the test sample and/or at least one comparison sample is biological matter from a Chlamydomonas reinhardtii algae.

In one embodiment the test sample is biological matter and/or at least one comparison sample from a non-vascular land plant, wherein the non-vascular plant is a bryophyte. In one embodiment, the test sample and/or at least one comparison sample is biological matter from a bryophyte selected from the group consisting of moss, liverwort and hornwort.

In one embodiment, the test sample and/or at least one comparison sample is biological matter from a hornwort.

In one embodiment, the test sample and/or at least one comparison sample is biological matter from a moss. In one embodiment, the test sample is biological matter from a moss of the *Physcomitrella* genus. In one embodiment, the test sample and/or at least one comparison sample is biological matter from a *Physcomitrella patens* or *Physcomitrella readeri* moss. In one embodiment, the test sample and/or at least one comparison sample is biological matter from a *Physcomitrella patens* moss.

In a preferred embodiment, the test sample and/or at least one comparison sample is biological matter from a liverwort. In one embodiment, the test sample and/or at least one comparison sample is biological matter from a plant of the Jungermanniopsida class. In one embodiment, the test sample and/or at least one comparison sample is biological matter from a plant of the Jungermanniidae or Metzgeriidae subclasses. In one embodiment, the test sample and/or at least one comparison sample is biological matter from a plant of the
Marchantiopsida class. In one embodiment, the test sample and/or at least one comparison sample is biological matter from a plant of the Marchantiidae or Sphaerocarpidae sublclasses. In one embodiment, the test sample and/or at least one comparison sample is biological matter from a plant of the Haplomitriopsida class.

In one embodiment, the test sample and/or at least one comparison sample is biological matter leafy liverwort, simple thalloid liverwort or a complex thalloid liverwort.

In one embodiment, the test sample and/or at least one comparison sample is biological matter from a plant of the Marchantia species. In one embodiment, the test sample and/or at least one comparison sample is biological matter from *Marchantia alpestris,*
*Marchantia aquatica, Marchantia berteroana, Marchantia carrii, Marchantia chenopoda, Marchantia debilis, Marchantia domingenis, Marchantia emarginata, Marchantia foliacia, Marchantia grossibarba, Marchantia inflexa, Marchantia linearis, Marchantia macropora, Marchantia novoguineensis, Marchantia paleacea, Marchantia palmata, Marchantia papillate, Marchantia pappeana, Marchantia polymorpha (also known* as *M. aquatica), Marchantia rubribarba, Marchantia solomonensis, Marchantia streimannii, Marchantia subgeminata, Marchantia vitiensis, Marchantia wallisii* or *Marchantia nepalensis.* In a preferred embodiment, the test sample and/or at least one comparison sample is biological matter from *Marchantia polymorpha.*

In one embodiment the test sample and/or at least one comparison sample is biological matter from a fern. In one embodiment the test sample and/or at least one comparison sample is biological matter from a Eusporangiate fern or a Leptosporangiate Ferns (also known as Polypodiidae ferns). In one embodiment the test sample and/or at least one comparison sample is biological matter from a fern. In one embodiment the test sample is biological matter from a Eusporangiate fern. In one embodiment, the test sample and/or at least one comparison sample is biological matter from a marattioid fern (Marattiidae, Marattiaceae), a horsetail fern (Equisetiidae, Equisetaceae), a whisk fern or a moonwort fern.

In one embodiment, the test sample and the at least one comparison sample are samples from independent plants of the same genus. In one embodiment, the test sample and the at least one comparison sample are samples from independent plants of the same species. In one embodiment, the test sample and the at least one comparison sample are samples from independent ferns of the same genus. In one embodiment, the test sample and the at least one comparison sample are samples from independent ferns of the same species. In one embodiment, the test sample and the at least one comparison sample are samples from independent algae of the same genus. In one embodiment, the test sample and the at least one comparison sample are samples from independent algae of the same species.

In one embodiment the reference DNA sequence is a known DNA sequence for a plant of the same genus as the plants used as the test sample and the at least one comparison sample. In one embodiment the reference DNA sequence is a known DNA sequence for a plant of the same species as the plants used as the test sample and the at least one comparison sample. In one embodiment the reference DNA sequence is a known DNA sequence for a fern of the same genus as the ferns used as the test sample and the at least one comparison sample. In one embodiment the reference DNA sequence is a known DNA sequence for a fern of the same species as the fern used as the test sample and the at least one comparison sample. In one embodiment the reference DNA sequence is a known DNA sequence for an algae of the same genus as the ferns used as the test sample and the at least one comparison sample. In one embodiment the reference DNA sequence is a known DNA sequence for an algae of the same species as the fern used as the test sample and the at least one comparison sample. In one embodiment, the method comprises (a) aligning the DNA sequence of a test sample to one or more reference DNA sequences and identifying a first set of sequence mismatches between the two sequences and/or (b) aligning the DNA sequence of at least one comparison sample to one or more reference DNA sequences and identifying a second set of sequence mismatches between the two sequences. Reference genomes are widely available on public databases and a skilled person understands how to select an appropriate reference sequence.

In one embodiment, the method comprises (a) aligning the DNA sequence of a test sample to two or more reference DNA sequences and identifying a first set of sequence mismatches; and/or (b) aligning the DNA sequence of at least one comparison sample to two or more reference DNA sequences and identifying a second set of sequence mismatches. In the case of multiple reference DNA samples (i.e. 2 or more), multiple independent comparisons are made. Firstly, between the DNA sequence of a test sample and the first reference DNA sequence to obtain a first set of mismatches. Secondly, between the test sample and the second reference DNA sequence to obtain a second set of mismatches. Thirdly, between the test sample and the reference DNA sequence to obtain a third set of mismatches etc. The first, second, third etc set of mismatches collectively form the first set of mismatches for step (a). Similarly, multiple independent comparisons are performed to compare the at least one comparison samples to the multiple reference DNA sequences to form the second set of mismatches for step (b).

In one embodiment the test sample and/or at least one comparison sample is biological matter from a sporulating organism, for example a sporulating plant, sporulating algae or sporulating fern. In one embodiment the test sample and/or at least one comparison sample is from a non-vascular plant that reproduces via spores. In one embodiment the test sample and/or at least one comparison sample is from a fern that reproduces via spores. In one embodiment the test sample and/or at least one comparison sample is from an algae that reproduces via spores.

In one embodiment, there is provided a method for identifying a mutation associated with a phenotype of interest in a liverwort plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent liverwort plants that have been exposed to a mutagen and wherein the independent liverwort plants exhibit the phenotype of interest and wherein the independent liverwort are the same genus; and
wherein the reference DNA sequence is a known reference sequence for a liverwort plant of the genus.

In one embodiment, there is provided a method for identifying a mutation associated with a phenotype of interest in a liverwort plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent liverwort plants that have been exposed to a mutagen and wherein the test sample is from a liverwort plant exhibiting the phenotype of interest and wherein the comparison sample is from an independent liverwort plant of the same genus that does not exhibit the phenotype of interest; and
wherein the reference DNA sequence is a known reference sequence for a liverwort plant of the genus.

In one embodiment, there is provided a method for identifying a mutation associated with a phenotype of interest in a fern, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent fern plants that have been exposed to a mutagen and wherein the independent fern plants exhibit the phenotype of interest and wherein the independent fern are the same genus; and
wherein the reference DNA sequence is a known reference sequence for a fern of the genus.

In one embodiment, there is provided a method for identifying a mutation associated with a phenotype of interest in a fern plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;
wherein the test sample and the comparison sample(s) are from independent fern plants that have been exposed to a mutagen and wherein the test sample is from a fern plant exhibiting the phenotype of interest and wherein the comparison sample is from an independent fern plant of the same genus that does not exhibit the phenotype of interest; and
wherein the reference DNA sequence is a known reference sequence for a fern of the genus.

In one embodiment, there is provided a method for identifying a mutation associated with a phenotype of interest in an algae, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent algae that have been exposed to a mutagen and wherein the independent algae exhibit the phenotype of interest and wherein the independent algae are the same genus; and
wherein the reference DNA sequence is a known reference sequence for an algae of the genus.

In one embodiment, there is provided a method for identifying a mutation associated with a phenotype of interest in an algae, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;
wherein the test sample and the comparison sample(s) are from independent algae that have been exposed to a mutagen and wherein the test sample is from an algae exhibiting the phenotype of interest and wherein the comparison sample is from an independent algae of the same genus that does not exhibit the phenotype of interest; and wherein the reference DNA sequence is a known reference sequence for an algae of the genus.

In one embodiment, the phenotype of interest is a morphological feature such as size, height, size, colour or structure of the non-vascular plant, fern or algae. In one embodiment, the phenotype of interest is an observable property such as increased yield, stress tolerance, stress resistance, abiotic stress tolerance, abiotic stress resistance, salt tolerance, salt resistance, sterility, drought resistance, drought tolerance, resistance to hot or cold temperatures, frost resistance, frost tolerance, growth rate, cell division rate, disease tolerance, disease resistance, disease sensitivity, herbicide tolerance, herbicide resistance, herbicide sensitivity, antibiotic tolerance, antibiotic resistance or antibiotic sensitivity. Dependent on the desired phenotype of interest, the plant, algae or fern may exhibit increased or decreased levels of the trait of interest (for example, herbicide resistance) compared to wild type plants. In one embodiment, the phenotype of interest is a plant exhibiting a 10, 20, 30, 40, 50, 60, 70, 80, 90 or 100% increase of the trait of interest compared to wild type levels. In one embodiment, the phenotype of interest is a plant exhibiting a 200, 250, 300, 350, 400, 450 or 500% increase of the trait of interest compared to wild type levels. In one embodiment, the phenotype of interest is a plant exhibiting a 10, 20, 30, 40, 50, 60, 70, 80, 90 or 100% decrease of the trait of interest compared to wild type levels.

In one embodiment, the phenotype of interest is herbicide resistance. In one embodiment, the phenotype of interest is herbicide tolerance. In one embodiment, the phenotype of interest is herbicide sensitivity. In one embodiment, the phenotype of interest may be increased resistance or increased tolerance to a viral, bacterial or fungal pathogen. In one embodiment, the phenotype of interest may be increased resistance or increased tolerance to a natural, synthetic or chemical herbicide. In a preferred embodiment, the phenotype of interest may be increased herbicide resistance. In a preferred embodiment, the phenotype of interest may be increased herbicide resistance to a specific herbicide. The test sample may be derived from a non-vascular plant resistant to a specific herbicide and the at least one comparison sample may be from independent non-vascular plants that are resistant to an alternative herbicide or displaying an alternative phenotype (i.e. not exhibiting resistance to the specific herbicide). In the case of herbicide resistance, resistance can be determined as survival of the plant following exposure to the herbicide of interest (i.e. the plant does not die following herbicide exposure). In one embodiment, survival is determined as survival of the plant at one week post herbicide exposure. In one embodiment, survival is determined as survival of the plant at two weeks post herbicide exposure. In one embodiment, survival is determined as survival of the plant at three weeks post herbicide exposure.

In one embodiment, the method comprises exposing a non-vascular plant, fern or algae or fern to an agent. Exposure to an agent can result in a plant, fern or algae exhibiting a phenotype of interest. The agent may be a nutrient, molecule that triggers a nutrient starvation response, plant growth modulator, plant growth inhibitor, plant growth enhancer, fertiliser or herbicide. In one embodiment, the test sample is from a non-vascular plant that has been exposed to an agent. In one embodiment, the test sample is from a fern that has been exposed to an agent. In one embodiment, the test sample is from an algae that has been exposed to an agent. In one embodiment, the test sample is from a non-vascular plant that has been exposed to a herbicide.

In one embodiment, the method comprises exposing the non-vascular plant to an agent of interest and selecting a non-vascular plant exhibiting a phenotype of interest based on the response of the non-vascular plant to the agent. In one embodiment, the method comprises exposing the non-vascular plant to a herbicide and selecting a non-vascular plant exhibiting a phenotype of interest based on the response of the non-vascular plant to the herbicide. In one embodiment, the method comprises exposing the non-vascular plant to a herbicide and selecting a non-vascular plant exhibiting herbicide resistance.

In one embodiment, the method comprises exposing the fern to an agent of interest and selecting a fern exhibiting a phenotype of interest based on the response of the fern to the agent. In one embodiment, the method comprises exposing the algae to an agent of interest and selecting an algae exhibiting a phenotype of interest based on the response of the algae to the agent.

In one embodiment, the method includes the following preliminary steps:
(i) exposing a population of non-vascular plants to a mutagen;
(ii) exposing a population of non-vascular plants to an agent;
(iii) obtaining a test sample from a mutagenized non-vascular plant;
(iv) obtaining at least one comparison sample from an independent mutagenized nonvascular plant; wherein (i) to (iv) are performed prior to (a).
Exposure to the agent can result in a phenotype of interest. In one embodiment, the method comprises exposing the non-vascular plant to an agent of interest and selecting a non-vascular plant exhibiting a phenotype of interest based on the response of the non-vascular plant to the agent.

In one embodiment, the method comprises the following preliminary steps:
(i) exposing a population of non-vascular plants to a mutagen;
(ii) exposing a population of non-vascular plants to a herbicide;
(iii) obtaining a test sample from a mutagenized non-vascular plant;
(iv) obtaining at least one comparison sample from an independent mutagenized nonvascular plant; wherein (i) to (iv) are performed prior to (a).

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants that have been exposed to a mutagen and further wherein the non-vascular plant has been exposed to an agent;
wherein the independent non-vascular plants exhibit the phenotype of interest and wherein the independent non-vascular plants are the same genus; and
wherein the reference DNA sequence is a known reference sequence for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants that have been exposed to a mutagen and further wherein the non-vascular plant has been exposed to an herbicide;
wherein the independent non-vascular plants exhibit the phenotype of interest and wherein the independent non-vascular plants are the same genus; and
wherein the reference DNA sequence is a known reference sequence for a non-vascular plant of the genus.

In one embodiment, there is provided a method for identifying a mutation associated with herbicide resistance in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample and the comparison sample(s) are from independent non-vascular plants that have been exposed to a mutagen and further wherein the non-vascular plant has been exposed to an herbicide;
and wherein the independent non-vascular plants exhibit resistance to the herbicide and wherein the independent non-vascular plants are the same genus; and
wherein the reference DNA sequence is a known reference sequence for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent non-vascular plants that have been exposed to a mutagen and further wherein the non-vascular plant has been exposed to an agent;
wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, there is provided a method for identifying a mutation associated with a phenotype of interest in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent non-vascular plants that have been exposed to a mutagen and further wherein the non-vascular plant has been exposed to an herbicide;
wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, there is provided a method for identifying a mutation associated with herbicide resistance in a non-vascular plant, wherein the method comprises:
(a) aligning the DNA sequence of a test sample to a reference DNA sequence and identifying a first set of sequence mismatches between the two sequences;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample and the comparison sample(s) are from independent non-vascular plants that have been exposed to a mutagen and wherein the test sample is from a non-vascular plant exhibiting herbicide resistance and wherein the comparison sample is from an independent non-vascular plant of the same genus that exhibits a different phenotype;
wherein the reference DNA sequence is a known reference sequence for a non-vascular plant of the genus.

In one embodiment, there is provided a method for identifying a mutation associated with a phenotype of interest in a non-vascular plant wherein the method comprises:
(i) exposing a population of non-vascular plants to a mutagen;
(ii) exposing a population of non-vascular plants to an agent;
(iii) obtaining a test sample from a mutagenized non-vascular plant exhibiting a phenotype of interest;
(iv) obtaining at least one comparison sample from an independent mutagenized nonvascular plant exhibiting the same phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are common to the first and second sets of mismatches, wherein the subset of mismatches are candidate mutations for the causative mutation;
wherein the reference DNA sequence is a known reference sequence for a non-vascular plant of the genus.

In one embodiment, there is provided a method for identifying a mutation associated with a phenotype of interest in a non-vascular plant wherein the method comprises:
(i) exposing a population of non-vascular plants to a mutagen;
(ii) exposing a population of non-vascular plants to a herbicide;
(iii) obtaining a test sample from a mutagenized non-vascular plant exhibiting a phenotype of interest;
(iv) obtaining at least one comparison sample from an independent mutagenized nonvascular plant exhibiting the same phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are common to the first and second sets of mismatches, wherein the subset of mismatches are candidate mutations for the causative mutation;
wherein the reference DNA sequence is a known reference sequence for a non-vascular plant of the genus.

In one embodiment, there is provided a method for identifying a mutation associated with herbicide resistance in a non-vascular plant wherein the method comprises:
(i) exposing a population of non-vascular plants to a mutagen;
(ii) exposing a population of non-vascular plants to a herbicide;
(iii) obtaining a test sample from a mutagenized non-vascular plant exhibiting resistance to the herbicide;
(iv) obtaining at least one comparison sample from an independent mutagenized nonvascular plant not exhibiting the same phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are common to the first and second sets of mismatches, wherein the subset of mismatches are candidate mutations for the causative mutation;
wherein the reference DNA sequence is a known reference sequence for a non-vascular plant of the genus.

In one embodiment, there is provided a method for identifying a mutation associated with a phenotype of interest in a non-vascular plant wherein the method comprises:
(i) exposing a population of non-vascular plants to a mutagen;
(ii) exposing a population of non-vascular plants to an agent;
(iii) obtaining a test sample from a mutagenized non-vascular plant exhibiting a phenotype of interest;
(iv) obtaining at least one comparison sample from an independent mutagenized nonvascular plant not exhibiting the phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are unique to the first set of mismatches, wherein the subset of mismatches are candidate mutations for the causative mutation;
wherein the reference DNA sequence is a known reference sequence for a non-vascular plant of the genus.

In one embodiment, there is provided a method for identifying a mutation associated with herbicide resistance in a non-vascular plant wherein the method comprises:
(i) exposing a population of non-vascular plants to a mutagen;
(ii) exposing a population of non-vascular plants to a herbicide;
(iii) obtaining a test sample from a mutagenized non-vascular plant exhibiting herbicide resistance;
(iv) obtaining at least one comparison sample from an independent mutagenized nonvascular plant not exhibiting the same phenotype of interest; and
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are unique to the first set of mismatches, wherein the subset of mismatches are candidate mutations for the causative mutation;
wherein the reference DNA sequence is a known reference sequence for a non-vascular plant of the genus.

In one embodiment, there is provided a method for identifying a mutation associated with herbicide resistance in a non-vascular plant wherein the method comprises:
(i) exposing a population of non-vascular plants to a mutagen;
(ii) exposing a population of non-vascular plants to a herbicide;
(iii) obtaining a test sample from a mutagenized non-vascular plant exhibiting herbicide resistance;
(iv) obtaining at least one comparison sample from an independent mutagenized nonvascular plant not exhibiting the same phenotype of interest and;
   (a) aligning the DNA sequence of the test sample to a reference DNA sequence and identifying a first set of sequence mismatches between the two sequences;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are unique to the first set of mismatches, wherein the subset of mismatches are candidate mutations for the causative mutation;
wherein the wherein the reference DNA sequence is a known reference sequence for a non-vascular plant of the genus.

In one embodiment, a method for identifying a mutation associated with a phenotype of interest in a non-vascular plant is provided, wherein the method comprises:
(i) obtaining genomic DNA from a test sample and genomic DNA from at least one comparison samples and generating a sequencing library;
(ii) performing cluster generation;
(iii) sequencing the genomic DNA from a test sample and genomic DNA from at least one comparison samples to obtain sequence reads;
   (a) aligning the DNA sequence of a test sample to a reference DNA sequence and identifying a first set of sequence mismatches between the two sequences;
      wherein the test sample is from a mutagenized non-vascular plant;
   (b) aligning the DNA sequence of at least one comparison sample to the reference DNA sequence and identifying a second set of sequence mismatches between the two sequences;
   (c) filtering the first set of mismatches with respect to the second set of mismatches to identify a subset of mismatches that are unique to the first set of mismatches, wherein the subset of mismatches are candidate mutations for the causative mutation;

wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, a method for identifying a mutation associated with a phenotype of interest in a non-vascular plant is provided, wherein the method comprises:
(i) obtaining genomic DNA from a test sample and genomic DNA from at least one comparison samples and generating a sequencing library;
(ii) performing cluster generation;
(iii) sequencing the genomic DNA from a test sample and genomic DNA from at least one comparison samples to obtain sequence reads;
   (a) aligning the DNA sequence of a test sample to a reference DNA sequence and identifying a first set of sequence mismatches between the two sequences;
      wherein the test sample is from a mutagenized non-vascular plant;
   (b) aligning the DNA sequence of at least one comparison sample to the reference DNA sequence and identifying a second set of sequence mismatches between the two sequences;
   (c) filtering the first set of mismatches with respect to the second set of sequence mismatches to identify a subset of mismatches that are common to the first and second sets of sequence mismatches;

wherein the test sample and the comparison sample(s) are from independent non-vascular plants exhibiting the phenotype of interest and wherein the independent non-vascular plants are the same genus; and
wherein the reference DNA sequence is a known reference sequence for a non-vascular plant of the genus.

In one embodiment, the method comprises sequencing the DNA sequences from the test sample and the at least one comparison sample to provide raw reads. In one embodiment, the step of sequencing the DNA comprises quality trimming and normalizing the raw read DNA sequences. Numerous software packages exist to perform each of these sequencing steps.

In one embodiment, the methods described herein are computer implemented methods. In one embodiment, method steps (a) to (c) and optionally (d) are executed by a computer program code. In one embodiment, a processor configured to execute computer program code stored in a computer readable medium executes method steps (a) to (c) and optionally (d) via the computer program code.

In one embodiment, the method comprises filtering the identified candidate mutations with biological filters to provide a reduced number of candidate mutations for the causative mutation. This further filtering step can be performed after the first filtering step (c) of filtering the first set of mismatches with respect to the second set of mismatches. The biological filter can be a filter for mutations not consistent with the mutational signature of the mutagenesis method (non-canonical mismatch filter). The biological filter can be a filter for mismatches that do not cause a change in the amino acid sequence of the coded protein. The biological filter can be a filter for non-coding mutations. The biological filter can be a filter for mismatches that are in the coding sequence of a gene with a predicted function.

In one embodiment a system for identifying a mutation associated with a phenotype of interest in a non-vascular plant is provided, wherein the system comprises a processor configured to execute computer program code stored in a computer readable medium, the computer program code configured to:
(a) align the DNA sequence of a test sample to a reference DNA sequence and identify a first set of sequence mismatches between the two sequences;
   wherein the test sample is from a mutagenized non-vascular plant;
(b) align the DNA sequence of at least one comparison sample to the reference DNA sequence and identify a second set of sequence mismatches between the two sequences;
(c) filtering the first set of mismatches with respect to the second set of mismatches to identify a subset of mismatches that are common to the first and second sets of mismatches, wherein the subset of mismatches are candidate mutations for the causative mutation;

wherein the test sample is from a non-vascular plant exhibiting the phenotype of interest and wherein the comparison sample is from an independent non-vascular plant of the same genus that does not exhibit the phenotype of interest; and
wherein the reference DNA sequence is a known reference sequence for a non-vascular plant of the genus.

In one embodiment, a system for identifying a mutation associated with a phenotype of interest in a non-vascular plant, wherein the system comprises a processor configured to execute computer program code stored in a computer readable medium, the computer program code configured to:
(a) align the DNA sequence of a test sample to a reference DNA sequence and identify a first set of sequence mismatches between the two sequences;
   wherein the test sample is from a mutagenized non-vascular plant;
(b) align the DNA sequence of at least one comparison sample to the reference DNA sequence and identify a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation;

wherein the test sample is from a non-vascular plant exhibiting the phenotype of interest and wherein the at least one comparison sample is from an independent non-vascular plant of the same genus that does not exhibit the phenotype of interest; and
wherein the reference DNA sequence is a known reference sequence for a non-vascular plant of the genus.

Other aspects are also envisaged such as, for example, a method for identification of a causative mutation causing a phenotype of interest in a test sample, the method comprising the step of selecting comparison samples based on either i) a complementation group prediction that the test sample and comparison samples of independent allelic M1 mutant lines form part of a complementation group and/or ii) a comparison causative mutation prediction that the comparison samples of independent non-allelic M1 mutant lines and M0 wild type plants do not harbour the causative mutation, wherein neither the
complementation group prediction or the comparison causative mutation prediction comprises a step of segregation analysis, and the method further comprising the steps of a processor configured to execute computer program code stored in a computer readable medium, the computer program code executing the method of: obtaining sample DNA sequence data associated with the test sample; obtaining reference DNA sequence data associated with a reference sample; obtaining comparison DNA sequence data associated with the comparison samples; determining a first set of mismatched DNA sequence data associated with the sample DNA sequence data and the reference DNA sequence data; determining further sets of mismatched DNA sequence data associated with the comparison DNA sequence data and the reference DNA sequence data; and filtering the first set of mismatched DNA sequence data with respect to the further sets of mismatched DNA sequence data to obtain a set of candidate mismatches that include the causative mutation for identification of the causative mutation from within the candidate mismatches.

As another example, also envisaged is a system for identification of a causative mutation causing a phenotype of interest in a test sample, the system comprising a means to select comparison samples based on either i) a complementation group prediction that the test sample and comparison samples of independent allelic M1 mutant lines form part of a complementation group and/or ii) a comparison causative mutation prediction that the comparison samples of independent non-allelic M1 mutant lines and M0 wild-type plants do not harbour the causative mutation, wherein neither the complementation group prediction or the comparison causative mutation prediction comprises a step of segregation analysis, and the system further comprising a processor configured to execute computer program code stored in a computer readable medium, the computer program code configured to: obtain sample DNA sequence data associated with the test sample; obtain reference DNA sequence data associated with a reference sample; obtain comparison DNA sequence data associated with the comparison samples; determine a first set of mismatched DNA sequence data associated with the sample DNA sequence data and the reference DNA sequence data; determine further sets of mismatched DNA sequence data associated with the comparison DNA sequence data and the reference DNA sequence data; and filter the first set of mismatched DNA sequence data with respect to the further sets of mismatched DNA sequence data to obtain a set of candidate mismatches that include the causative mutation for identification of the causative mutation from within the candidate mismatches.

In one aspect, a method for identification of a causative mutation causing a phenotype of interest in a test sample is provided, the method comprising the step of selecting comparison samples based on either i) a prediction that the test sample and comparison samples form part of a complementation group and/or ii) a prediction that the comparison samples do not harbour the causative mutation, and the method further comprising the steps of a processor configured to execute computer program code stored in a computer readable medium, the computer program code executing the method of:
obtaining a sample DNA sequence data associated with the test sample;
obtaining a reference DNA sequence data associated with a reference sample;
obtaining a comparison DNA sequence data associated with the comparison samples;
determining a first set of mismatched DNA sequence data associated with the sample DNA sequence data and the reference DNA sequence data;
determining further sets of mismatched DNA sequence data associated with the comparison DNA sequence data and the reference DNA sequence data; and filtering the first set of mismatched DNA sequence data with respect to the further sets of mismatched DNA sequence data to obtain a set of candidate mismatches that include the causative mutation for identification of the causative mutation from within the candidate mismatches.

In one embodiment, the comparison samples are selected based on the prediction that the test sample and comparison samples form part of a complementation group, and further comprising the computer program code executing the method of:
filtering the first set of mismatched DNA sequence data with respect to the further sets of mismatched DNA sequence data by:
determining common mismatched DNA sequence data that is in both of i) the first set of mismatched DNA sequence data and ii) the further sets of mismatched DNA sequence data, and
generating the set of candidate mismatches based on the common mismatched DNA sequence data.

In one embodiment, the comparison samples are selected based on the prediction that the comparison samples do not harbour the causative mutation, and further comprising the computer program code executing the method of:
filtering the first set of mismatched DNA sequence data with respect to the further sets of mismatched DNA sequence data by:
determining uncommon mismatched DNA sequence data that is in the first set of mismatched DNA sequence data and not in the further sets of mismatched DNA sequence data and
generating the set of candidate mismatches based on the uncommon mismatched DNA sequence data.

In one embodiment, the step of determining the first set of mismatched DNA sequence data comprises the steps of the computer program code:
aligning each read of the sample DNA sequence data with the reference DNA sequence data; and
sorting the aligned reads of the sample DNA sequence data based on the position of the reads of the sample DNA sequence data with respect to the reference DNA sequence data.

In one embodiment, the step of determining the second or further sets of mismatched DNA sequence data comprises the steps of the computer program code:
aligning each read of the comparison DNA sequence data with the reference DNA sequence data; and
sorting the aligned reads of the comparison DNA sequence data based on the position of the reads of the comparison DNA sequence data with respect to the reference DNA sequence data.

In one embodiment, the step of determining the first set of mismatched DNA sequence data comprises the steps of the computer program code:
rejecting at least one region of the sample DNA sequence data that aligns with the reference DNA sequence data based on the size of the region that aligns being over a predetermined sequencing depth.

In one embodiment, the step of determining the second or further sets of mismatched DNA sequence data comprises the steps of the computer program code:
rejecting at least one region of the comparison DNA sequence data that aligns with the reference DNA sequence data based on the size of the region that aligns being over a predetermined sequencing depth.

In one embodiment, wherein the computer program code further executes the method of:
determining a plurality of first sets of mismatched DNA sequence data,
determining a number of sets in the plurality of first set in which a mismatch occurs, and
upon a positive determination that the number of sets in which the mismatch occurs exceeds a predetermined threshold, adding the mismatched DNA sequence data associated with the mismatch to the set of candidate mismatches.

In one aspect, a system for identification of a causative mutation causing a phenotype of interest in a test sample is provided, the system comprising a means to select comparison samples based on either i) a prediction that the test sample and comparison samples form part of a complementation group and/or ii) a prediction that the comparison samples do not harbour the causative mutation, and the system further comprising a processor configured to execute computer program code stored in a computer readable medium, the computer program code configured to:
obtain a sample DNA sequence data associated with the test sample;
obtain a reference DNA sequence data associated with a reference sample;
obtain a comparison DNA sequence data associated with the comparison samples;
determine a first set of mismatched DNA sequence data associated with the sample DNA sequence data and the reference DNA sequence data;
determine further sets of mismatched DNA sequence data associated with the comparison DNA sequence data and the reference DNA sequence data; and
filter the first set of mismatched DNA sequence data with respect to the further sets of mismatched DNA sequence data to obtain a set of candidate mismatches that include the causative mutation for identification of the causative mutation from within the candidate mismatches.

In one embodiment, the comparison samples are selected based on the prediction that the test sample and comparison samples form part of a complementation group, and the computer program code is further configured to:
filter the first set of mismatched DNA sequence data with respect to the further sets of mismatched DNA sequence data by the computer program code being configured to:
determine common mismatched DNA sequence data that is in both of i) the first set of mismatched DNA sequence data and ii) the further sets of mismatched DNA sequence data, and
generate the set of candidate mismatches based on the common mismatched DNA sequence data.

In one embodiment, the comparison samples are selected based on the prediction that the comparison samples do not harbour the causative mutation, and the computer program code is further configured to:
filter the first set of mismatched DNA sequence data with respect to the further sets of mismatched DNA sequence data by the computer program code being configured to:
determine uncommon mismatched DNA sequence data that is in the first set of mismatched DNA sequence data and not in the further sets of mismatched DNA sequence data and
generate the set of candidate mismatches based on the uncommon mismatched DNA sequence data.

In one embodiment, to determine the first set of mismatched DNA sequence data the computer program code is further configured to:
align each read of the sample DNA sequence data with the reference DNA sequence data; and
sort the aligned reads of the sample DNA sequence data based on the position of the reads of the sample DNA sequence data with respect to the reference DNA sequence data.

In one embodiment, to determine the second or further sets of mismatched DNA sequence data the computer program code is further configured to:
align each read of the comparison DNA sequence data with the reference DNA sequence data; and
sort the aligned reads of the comparison DNA sequence data based on the position of the reads of the comparison DNA sequence data with respect to the reference DNA sequence data.

In one embodiment, to determine the first set of mismatched DNA sequence data the computer program code is further configured to:
reject at least one region of the sample DNA sequence data that aligns with the reference DNA sequence data based on the size of the region that aligns being over a predetermined sequencing depth.

In one embodiment, to determine the further sets of mismatched DNA sequence data the computer program code is further configured to:
reject at least one region of the comparison DNA sequence data that aligns with the reference DNA sequence data based on the size of the region that aligns being over a predetermined sequencing depth.

In one embodiment, the computer program code is further configured to:
determine a plurality of first sets of mismatched DNA sequence data,
determine a number of sets in the plurality of first set in which a mismatch occurs, and
upon a positive determination that the number of sets in which the mismatch occurs exceeds a predetermined threshold, add the mismatched DNA sequence data associated with the mismatch to the set of candidate mismatches.

In one aspect, a computer readable storage medium having a computer program recorded therein is provided, the program being executable by a computer apparatus to make the computer perform the method of any one of the embodiments disclosed herein.

### Industrial Applicability

The arrangements described are applicable to the DNA sequencing industries and particularly for industries dealing with the detection of causative mutations in DNA sequences.

The disclosed software method and/or system enables the discovery of causative mutations without the need to cross mutants. Therefore, causative mutations in sterile mutants may be identified using the disclosed software method and/or system. Identifying mutations that cause sterility may have applications in the field of agricultural technologies. Mutations causing sterility may be engineered in agriculturally relevant plant species to produce sterile seeds. For example, mutations causing sterility may be applied to Gene Use Restriction Technology (GURT).

The foregoing describes only some embodiments of the present invention, and modifications and/or changes can be made thereto without departing from the scope and spirit of the invention, the embodiments being illustrative and not restrictive.

In the context of this specification, the word "comprising" means "including principally but not necessarily solely" or "having" or "including", and not "consisting only of". Variations of the word "comprising", such as "comprise" and "comprises" have correspondingly varied meanings.

### Example 1: Discovery of a mutation in the RHO GTPASES of PLANTS ENHANCER PROTEIN gene impairing fertility (case B)

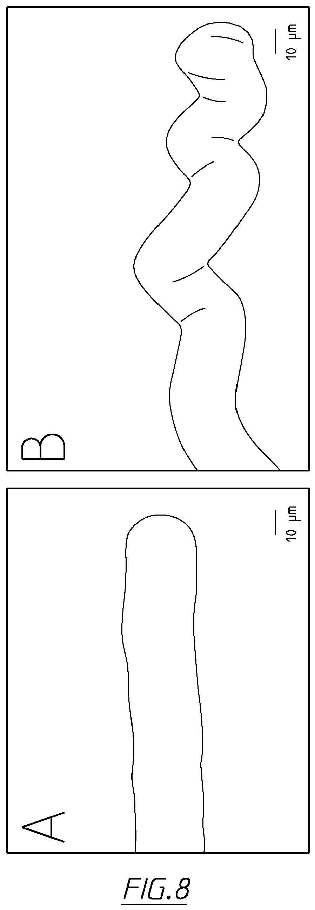

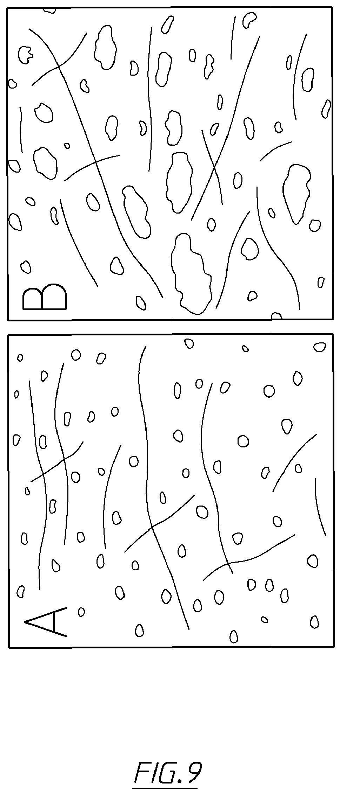

Several independent mutant lines were generated by irradiating *Marchantia polymorpha* spores with ultraviolet B. Mutants lines were classified into two phenotypic groups: some had straight rhizoids (Fig. 8A) and intact epidermis (Fig. 9A), some had wavy rhizoids (Fig. 8B) and stretched epidermis (Fig. 9B).

We aimed to identify the causative mutation in the UV4.32 mutant line, which has wavy rhizoids and stretched epidermis. DNA was extracted from a UV4.32 mutant with wavy rhizoids and stretched epidermis using the whole plant as a sample and standard DNA PhenolChlorophorm-IAA extraction. The genomes of UV4.32 and the genome of 7 independent mutant lines with straight rhizoids and intact epidermis were sequenced using Illumina's HiSeq-2000 platform technology.

Raw reads were quality trimmed using Trimmomatic-0.32 and normalised using Khmer0.7.1 with a k-mer size of 31. Resulting reads were aligned against the reference genome using bowtie2-2.1.0 set in --very-sensitive-local mode. The reference genome used is a draft Marchantia polymorpha genome assembly publicly available on the NCBI Whole Genome Shotgun (WGS) database.

Alignments were position sorted and mismatches within reads with q quality higher than 35 were extracted using the function sort and mpileup from bio-samtools-2.0.5. Because they were likely caused by misalignments, mismatches in regions with coverage exceeding
100X were excluded using the varFilter function from bcftools of the samtools-0.1.9 package. Then, mismatches were retained only if they were supported by more than 7 reads and if they appeared sufficiently homozygous based on a negative FQ value or AF1 value higher than
0.5001.

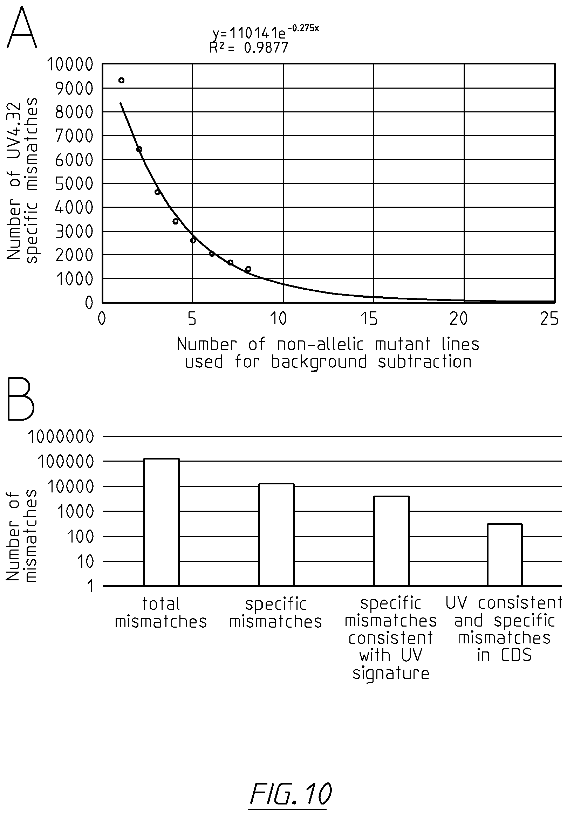

In total, 143 292 mismatches were identified in UV4.32 before any filtering. The number of mismatches specific to UV4.32 decreased with the number of UV mutant lines with straight rhizoids and intact epidermis used for filtering (Fig. 10A).

Ultimately, using all filtering lines sequenced, the number of candidate mismatches was reduced to 12 000 mismatches, or more than 90% decrease (Fig. 3B). This shows that filtering step of subtracting the set of mismatches in the test sample by the set of mismatches in comparison samples that are predicted not to harbour the causative mutation increased the stringency of candidate mismatches identification, prior to standard filtering steps.

Subsequent filtering steps were performed to filter for mismatches inconsistent with UV signature, filter for mismatches outside the gene coding sequence and to filter for nonsynonymous mismatches. These three filtering steps further reduced the number of candidate mismatches to 10 mutations that were consistent with the expected UV mutation signature (Fig. 3), were predicted to be in the coding sequence of a gene (Fig. 3) and to change the amino acid sequence of the corresponding protein (Table 1).

**Table 1: Candidate mutations for UV4.32 in Marchantia genes and corresponding Arabidopsis homologous genes. Arabidopsis thaliana is the most established model for plant genetics and the function of Marchantia polymorpha genes may be inferred by analogy with the function of Arabidopsis genes.**

| **Mutated gene model** | **Arabidopsis homolog** | **Annotation of Arabidopsis homolog** | **Type of mutation** |
|---|---|---|---|
| MpREN | AT5G12150/PHGAP1 | Rho GTPase Activating Protein | Frame shift, early stop |
| Mp1660s1160 | AT5G38840 | SMAD/FHA domain containing protein | missense |
| Mp2415s1240 | AT2G29510 | hypothetical protein DUF3527 | missense |
| Mp2490s1660 | AT1G74410 | RING protein | missense |
| Mp2782s1160 | None | NA | missense |
| Mp3036s1070 | AT3G54750 | unannotated | missense |

| Mp3802s1070 | AT1G06560 | NOP2C, RNA methylation | missense |
|---|---|---|---|
| Mp4605s1070 | None | NA | missense |
| Mp773s1730 | AT1G73060 | LPA3 | missense |
| Mp909s1190 | A T2G07360 | SH3 domain-containing protein | missense |

Of the 10 mutations, the strongest mutation is a 2 base pair deletion causing an early stop codon in Mp*REN* (Table 1). *Ren* mutants are known to exhibit the same phenotype as UV4.32 (Honkanen et al, 2016 and unpublished data/Fig2B). This suggests that the subsequent filtering steps were sufficiently conservative.

Altogether, this shows that the version of our pipeline based on subtracting the set of mismatches in the test sample by the set of mismatches in comparison samples that are predicted not to harbour the causative mutation enables the identification of a small number of mutations, including the causative mutation, without needing to outcross the mutant lines.

### Example 2: Discovery of mutations in the ACETOLACTATE SYNTHASE gene causing chlorsulfuron resistance (case A)

*Marchantia polymorpha* spores were irradiated with ultraviolet B irradiation and seven independent mutant lines resistant to the herbicide chlorsulfuron were identified. Chlorsulfuron resistance was determined by a *Marchantia polymorpha* plant that was alive two weeks following exposure to a lethal dose of Chlorsulfuron (0.1 ppm dose, i.e. a dose sufficient to kill 100% of wild-type plants).

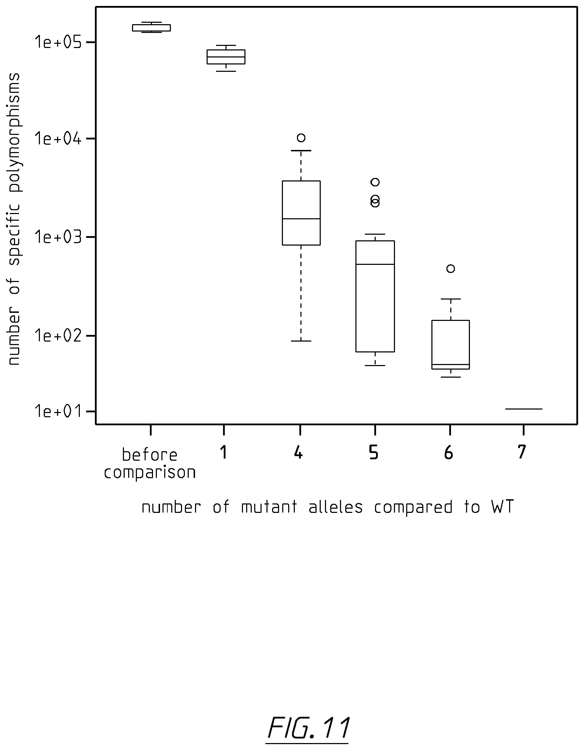

Since all mutant plants shared the same phenotype - chlorsulfuron resistance - we hypothesised that they each harbour the same causative mutation. Comparing the chlorsulfuron resistant mutants to the reference genome individually identified over 100 000 mismatches and we first filtered our mismatches that were also present in a M0 wild type genome (Fig. 11, the 2 left-most scatter boxes).

To test the efficiency of the allelism-based version of our pipeline, we applied it to combinations of 4, 5, 6, and all 7 chlorsulfuron mutants. The more allelic subtracting lines we use, the more efficient the pipeline becomes. In fact, using all 7 chlorsulfuron resistant lines, we decreased the number of mismatches from nearly 100 000 to 11 candidate mutations that are consistent with the expected mutational signature and are in the coding sequence of a gene (Fig. 11).

Of the 11 candidate mutations that are common to all 7 chlorsulfuron resistant mutants but absent from wild type, 5 cause a change in the amino acid sequence of the coded protein (Table 3). Of those 5 candidate mutations, only one is in a gene with a predicted function. In fact, this exact mutation in the acetolactate synthase gene is known to cause chlorsulfuron resistance in other plant models.

**Table 3: Candidate mutations for chlorsulfuron mutants (case A)**

| Mutated gene model | Arabidopsis homolog | Annotation of Arabidopsis homolog | Type of mutation |
|---|---|---|---|
| Mp3229s1050 | None | NA | nonsense |
| Mp2743s1010 | None | NA | nonsense |
| Mp3364s1000 | None | NA | missense |
| Mp4485s1300 | None | NA | missense |
| Mp2116s1050 | A T3G42690 | Acetolactate synthase | missense |

### Example 3: Discovery of mutations in the ACETOLACTATE SYNTHASE gene causing chlorsulfuron resistance (case AB)

To improve the power of the pipelines exemplified in Example 1 and Example 2, we combined both approaches: in this embodiment of the pipeline, the causative mutations is looked for in the group of mismatches that are common to allelic mutants and absent from wild type and non-allelic mutants.

Using 3 chlorsulfuron sensitive mutagenized lines, we filtered 4 of the 11 chlorsulfuronresistant specific mismatches previously identified as being consistent with the expected mutational signature and being in the coding sequence of a gene, finally leaving us with only 4 candidate mutations (Table 4) predicted to cause a change in the amino acid sequence of a protein.

This represents a 20-30% increase in the power of the pipeline compared the pipeline exemplified in Example 2 alone. Because the power of the pipeline in Example 1 and 2 increases with the number of allelic and non-allelic subtracting lines resp., we predict that the power of the pipeline exemplified in the present Example will increase further if we use more allelic and non-allelic subtracting lines.

**Table 4. Candidate mutations (Table 4) predicted to cause a change in the amino acid sequence of a protein.**

| Mutated gene model | Arabidopsis homolog | Annotation of Arabidopsis homolog | Type of mutation |
|---|---|---|---|
| Mp2743s1010 | None | NA | nonsense |
| Mp3364s1000 | None | NA | missense |
| Mp4485s1300 | None | NA | missense |
| Mp2116s1050 | A T3G42690 | Acetolactate synthase | missense |

## Claims

1. A computer-implemented method for identifying a mutation associated with a phenotype of interest in a nonvascular plant, wherein the method comprises:
(a) aligning the DNA sequence of a test sample to a reference DNA sequence and identifying a first set of sequence mismatches between the two sequences; wherein the test sample is from a mutagenized non-vascular plant;
(b) aligning the DNA sequence of at least one comparison sample to the reference DNA sequence and identifying a second set of sequence mismatches between the two sequences;
(c) filtering the first set of mismatches with respect to the second set of mismatches to identify a subset of mismatches that are unique to the first set of mismatches, wherein the subset of mismatches are candidate mutations for the causative mutation; wherein the test sample is from a non-vascular plant exhibiting the phenotype of interest;
wherein the at least one comparison sample is from an independent non-vascular plant of the same genus that does not exhibit the phenotype of interest; and wherein the reference DNA sequence is a known reference sequence for a non-vascular plant of the genus.

2. A computer-implemented method for identifying a mutation associated with a phenotype of interest in a nonvascular plant, wherein the method comprises:
(a) aligning the DNA sequence of a test sample to a reference DNA sequence and identifying a first set of sequence mismatches between the two sequences; wherein the test sample is from a mutagenized non-vascular plant;
(b) aligning the DNA sequence of at least one comparison sample to the reference DNA sequence and identifying a second set of sequence mismatches between the two sequences;
(c) filtering the first set of mismatches with respect to the second set of sequence mismatches to identify a subset of mismatches that are common to the first and second sets of sequence mismatches;
wherein the test sample and the comparison sample(s) are from independent non-vascular plants exhibiting the phenotype of interest and wherein the independent non-vascular plants are the same genus; and wherein the reference DNA sequence is a known reference sequence for a non-vascular plant of the genus.

3. The computer-implemented method of claim 1, wherein the method further comprises:
(b-i) aligning the DNA sequence of at least one additional comparison sample to the reference DNA sequence and identifying a third set of sequence mismatches between the two sequences; wherein the additional comparison sample(s) are from independent non-vascular plants exhibiting the phenotype of interest and wherein the independent non-vascular plants are the same genus: and wherein
(c) further comprises filtering the first set of mismatches with respect to the third set of sequence mismatches to identify a subset of mismatches that are common to the first and second sets of sequence mismatches, wherein the two subsets of mismatches are candidate mutations for the causative mutation.

4. The computer-implemented method of any one of claims 1 to 3, wherein the phenotype of interest is an observable property selected from the group consisting of increased yield, stress tolerance, stress resistance, abiotic stress tolerance, abiotic stress resistance, salt tolerance, salt resistance, sterility, drought resistance, drought tolerance, resistance to hot or cold temperatures, frost resistance, frost tolerance, plant growth rate, plant cell division rate, disease tolerance, disease resistance, disease sensitivity, herbicide tolerance, herbicide tolerance, herbicide sensitivity, antibiotic tolerance, antibiotic resistance and antibiotic sensitivity.

5. The computer-implemented method of any one of claims 1 to 3, wherein the phenotype of interest is increased resistance or increase tolerance to a naturel, synthetic or chemical herbicide.

6. The computer-implemented method of any one of claims 1 to 5, wherein the non-vascular plant is a leafy liverwort, simple thalloid liverwort or a complex thalloid liverwort; optionally wherein the non-vascular plant is selected from the group consisting of *Marchantia alpestris, Marchantia aquatica, Marchantia berteroana, Marchantia carrii, Marchantia chenopoda, Marchantia debilis, Marchantia domingenis, Marchantia emarginata, Marchantia foliacia, Marchantia grossibarba, Marchantia inflexa, Marchantia linearis, Marchantia macropora, Marchantia novoguineensis, Marchantia paleacea, Marchantia palmata, Marchantia papillate, Marchantia pappeana, Marchantia polymorpha, Marchantia rubribarba, Marchantia solomonensis, Marchantia streimannii, Marchantia subgeminata, Marchantia vitiensis, Marchantia wallisii* and *Marchantia nepalensis.*

7. The computer-implemented method of any one of claims 1 to 6, wherein the mutagenized test sample is a M1 mutant; optionally wherein the comparison sample from an independent non-vascular plant is a mutagenized non-vascular plant.

8. The computer-implemented method of any one of claims 1 to 7, wherein the mutagenized test sample comprises a non-naturally occurring mutation.

9. The computer-implemented method of any one claims 1 to 8, wherein the method does not comprise a step of segregation analysis, complex segregation analysis or bulk segregation analysis; optionally wherein the method does not require a step of self-fertilisation, fertilization, outcrossing, back-crossing or fertilization with a near-isogenic line of the non-vascular plants.

10. The computer-implemented method of any one of claims 1 to 9, wherein step (b) comprises aligning the DNA sequence of 2, 3, 4, 5, 6, 7, 8, 9, 10, 11, 12, 13, 14, 15 or more comparison samples to the reference DNA sequence and identifying a second set of sequence mismatches between the two sequences; optionally and/or wherein the method comprises (d) further filtering the candidate mutations with biological filters.

11. The computer-implemented method of any one of claims 1 to 10, wherein the non-vascular plant is a haploid.

12. The computer-implemented method of any one of claims 1 to 11, wherein the non-vascular plant is selected from the group consisting of moss, liverwort and hornwort; optionally wherein the non-vascular plant is a moss selected from *Physcomitrella patens* or *Physcomitrella readeri.*

13. The computer-implemented method of any one of claims 1 to 12, wherein the phenotype of interest is a morphological feature selected from the group consisting of plant size, plant height, leaf size, plant colour and plant structure.

14. The computer-implemented method of any one of claims 1 to 13, wherein the phenotype of interest is increased resistance or increased tolerance to a viral, bacterial or fungal pathogen.

15. The computer-implemented method of any one of claims 1 to 14, wherein the method further comprises:
(i) exposing a population of non-vascular plants to a mutagen;
(ii) exposing a population of non-vascular plants to an agent;
(iii) obtaining a test sample from a mutagenized non-vascular plant;
(iv) obtaining at least one comparison sample from an independent mutagenized nonvascular plant; wherein (i) to (iv) are performed prior to (a).
